# EUROPÄISCHE PATENTSCHRIFT

(11) **EP 4 309 774 B1**
(45) Veröffentlichungstag und Bekanntmachung des Hinweises auf die Patenterteilung: **03.09.2025**
(21) Anmeldenummer: 22185605.7
(22) Anmeldetag: 19.07.2022
(51) Int. Cl.: B01F 33/501, B01F 35/71, B01F 35/75, B01F 101/20, A61B 17/88

(54) **VORRICHTUNG UND VERFAHREN ZUM BEREITSTELLEN EINES KNOCHENZEMENTTEIGS**
METHOD AND DEVICE FOR PROVIDING A BONE CEMENT MASS
DISPOSITIF ET PROCÉDÉ DE FOURNITURE D'UNE PÂTE DE CIMENT OSSEUX

(43) Veröffentlichungstag der Anmeldung: 24.01.2024
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(56) Entgegenhaltungen:
- EP-A1- 3 100 694
- EP-A1- 3 643 398
- EP-A2- 1 016 452
- DE-A1- 102018 131 266
- US-A1- 2003 067 837

## Beschreibung

Die Erfindung betrifft eine Vorrichtung zum Bereitstellen eines Knochenzementteigs aus zwei Ausgangskomponenten, umfassend
eine Mischeinheit aufweisend eine hohlzylinderförmige Kartusche mit einem Innenraum, wobei in dem Innenraum ein axial im Innenraum beweglicher Austragskolben angeordnet ist, welcher den Innenraum in einen proximalen Teil des Innenraums und einen distalen Teil des Innenraums unterteilt, wobei der proximale Teil und der distale Teil des Innenraums über ein Leitungsmittel fluidleitend miteinander verbunden sind,
wobei im proximalen Teil des Innenraums ein Knochenzementpulver als erste Ausgangskomponente lagert und wobei im distalen Teil des Innenraums ein axial im Innenraum beweglicher Förderkolben angeordnet ist,
und ein Reservoir für eine Monomerflüssigkeit als zweite Ausgangskomponente, welches über einen Einlasskanal mit dem distalen Teil des Innenraums zum Einleiten der Monomerflüssigkeit aus dem Reservoir in die Mischeinheit fluidleitend verbunden oder verbindbar ist.

Die Erfindung betrifft weiterhin ein Verfahren zum Bereitstellen eines Knochenzementteigs aus zwei Ausgangskomponenten mittels einer derartigen Vorrichtung.

### Hintergrund der Erfindung

Es werden erhebliche Bemühungen unternommen, Vorrichtungen und Verfahren zum Bereitstellen von Knochenzement aufzuzeigen, mittels derer Knochenzementteig einfach, zuverlässig und schnell bereitgestellt werden kann. Ein wichtiger Aspekt bei der Bereitstellung von Knochenzementteig ist die Vermeidung von Lufteinschlüssen, wie beispielsweise Gasblasen, im Knochenzement. Zu deren Vermeidung wurden eine Vielzahl von Vakuum-Zementiersystemen beschreiben, von denen exemplarisch folgende genannt sind: US 6,033,105 A, US 5,624,184 A, US 4,671,263 A, US 4,973,168 A, US 5,100,241 A, WO 99/67015 A1, EP 1020167 A2, US 5,586,821 A, EP 1016452 A2, DE 3640279 A1, WO 94/26403 A1, EP 1005901 A2, EP 1886647 A1, US 5,344,232 A.

Es besteht im Markt der Wunsch zur Vereinfachung der Bereitstellung von Knochenzementteig. Eine Weiterentwicklung besteht in der Entwicklung von Zementiersystemen, in denen beide Ausgangskomponenten in separaten Bereichen der Mischsysteme gelagert sind und erst unmittelbar vor der Zementierapplikation im Zementiersystem miteinander vermischt werden.

Solche geschlossenen, sogenannten Full-Prepacked-Systeme, sind beispielsweise in folgenden Schriften genannt: EP 0 692 229 A1, DE 10 2009 031 178 B3, US 5,997,544 A, US 6,709,149 B1, DE 698 12 726 T2, EP 0 796 653 A2, US 5,588,745 A.

In den vorgenannten Full-Prepacked-Systemen erfolgt die Vermischung einer Monomerflüssigkeit mit einem Knochenzementpulver durch mechanisches Vermischen, beispielsweise mittels eines Mischstabes.

In der Patentschrift EP 3 320 870 B1 wird im Gegensatz zu den vorgenannten Full-Prepacked-Systemen eine Vorrichtung beschrieben, bei welcher die Vermischung einer Monomerflüssigkeit mit einem Knochenzementpulver lediglich durch Einpressen der Monomerflüssigkeit in, insbesondere verdichtetes, Knochenzementpulver erfolgt. Die beschriebene Vorrichtung kommt daher ohne mechanisches Vermischen, insbesondere ohne einen Mischstab, aus. Derartige Vorrichtungen sind infolgedessen mischeinrichtungsfrei ausgestaltet.

In der Vorrichtung ist ein mit Monomerflüssigkeit gefüllter Behälter axial hinter einem mit einem Knochenzementpulver gefülltem Bereich innerhalb einer Kartusche lagert. Zwischen Knochenzementpulver und Behälter ist ein Austragskolben angeordnet. Um einen Knochenzementteig bereitzustellen, wird ein Förderkolben, welcher auf einer dem Austragskolben gegenüberliegenden Seite des Behälters angeordnet ist, in Richtung des Austragskolbens vorgetrieben, wodurch es zu einem Öffnen des Behälters, insbesondere durch teilweises Zerbersten eines Behälters in Form einer Glasampulle in Behälterteilstücke, kommt. Die aus dem Behälter austretende Monomerflüssigkeit wird durch fortgeführtes Vortreiben des Förderkolbens in das Knochenzementpulver unter Ausbildung des Knochenzementteigs gefördert.

Vergleichbare Vorrichtungen sind ebenso in den Patentschriften EP 3 320 869 B1 und EP 3 403 716 B1 beschrieben.

Ein Nachteil dieser Vorrichtungen besteht darin, dass zum Öffnen und Fördern der Monomerflüssigkeit in das Knochenzementpulver der Behälter im Wesentlichen vollständig zerstört werden muss, was zum einen eine vergleichsweise hohe Kraftanstrengung bei einem Anwender der Vorrichtung vonnöten macht und zum anderen ein im Wesentlichen vollständiges Fördern der Monomerflüssigkeit in das Knochenzementpulver, insbesondere aufgrund der Bruchstücke des Behälters, erschwert.

In der Patentanmeldung EP 3 838 391 A1 wird ebenso eine mischeinrichtungsfreie Vorrichtung zur Bereitstellung eines Knochenzementteigs beschrieben.

Bei dieser Vorrichtung lagert ein mit einer Monomerflüssigkeit gefüllter Behälter in einem Reservoir, von welchem die Monomerflüssigkeit nach dem Öffnen des Behälters über ein Leitungsmittel in einen distalen Teil eines Innenraums fließen kann, bevor sie durch Vortreiben eines Kolbens in ein Knochenzementpulver in einem proximalen Teil des Innenraums förderbar ist. Das Reservoir ist dabei so angeordnet, dass der Behälter weder von dem Kolben geöffnet, noch beim Fördern der Monomerflüssigkeit in das Knochenzementpulver zusammengedrückt wird.

Ein Nachteil dieser Vorrichtung ist, dass die Monomerflüssigkeit durch das Leitungsmittel lediglich langsam und/oder stoßweise in den distalen Teil des Innenraums fließen kann, da der Durchmesser des Leitungsmittels so ausgestaltet sein muss, dass der Behälter, oder Teilstücke davon, nicht durch das Leitungsmittel in den Innenraum gelangen können. Weiterhin kann durch das Leitungsmittel nur erschwert ein Gas, welches durch die in den Innenraum einfließende Monomerflüssigkeit von dort verdrängt wird, ausgeleitet werden. Das Leitungsmittel ist daher nicht für einen guten Stoffaustausch zwischen Reservoir in Innenraum ausgestaltet. Dies ist von Nachteil, da insbesondere bei zeitkritischen Operationen ist eine schnelle, sichere und im Wesentlichen vollständige Bereitstellung der Monomerflüssigkeit zur Anmischung des Knochenzementteigs vonnöten ist. Ein weiterer Nachteil der Vorrichtung ist deren recht komplexer Aufbau mit vielen beweglichen Bauteilen. Zudem ist der Kolben, welcher bereits zum Fördern der Monomerflüssigkeit in das Knochenzementpulver benutzt wurde, nicht zum Austragen des Knochenzementteigs aus der Vorrichtung ausgelegt. Insbesondere würde dabei auch das von der Vorrichtung abstehende Reservoir das Austragen des Knochenzementteigs aus der Vorrichtung aus sterischen Gründen erschweren.

Es besteht daher im Markt der Wunsch zur weiteren Vereinfachung von Vorrichtungen zum Bereitstellen von Knochenzementteig.

EP 3 643 398 A1 offenbart eine Vorrichtung zum Bereitstellen eines Knochenzementteigs entsprechend den Oberbegriffen der Ansprüche 1, 2.

### Aufgaben

Eine Aufgabe der vorliegenden Erfindung ist es, einen oder mehrere der sich aus dem Stand der Technik ergebenen Nachteile zumindest teilweise zu überwinden.

Im Speziellen basiert die Erfindung auf dem Ziel, eine Vorrichtung zur Verfügung zu stellen, welche ein einfaches und anwendungssicheres Öffnen einer oder mehrerer Ampullen, insbesondere Glasampullen, mit einer Monomerflüssigkeit zum einfachen, schnellen und anwendungssicheren Bereitstellen eines Knochenzementteigs erlaubt. Insbesondere soll das Öffnen der Ampulle oder der Ampullen mit möglichst wenig Aufwand und unter Vermeidung zusätzlicher, separater Werkzeuge erfolgen. Weiterhin soll das Öffnen der Ampulle mit möglichst wenigen Bauteilen ermöglicht sein. Weiterhin soll die Monomerflüssigkeit möglichst verlustfrei und schnell zum Bereitstellen des Knochenzementteigs zur Verfügung stehen. Das Fördern der Monomerflüssigkeit in ein Knochenzementpulver zur Bereitstellung des Knochenzementteigs soll mit möglichst geringem Kraftaufwand durchführbar sein.

Die Vorrichtung soll den Knochenzementteig ohne eine mechanische Durchmischung der Ausgangskomponenten bereitstellen. Die Vorrichtung soll ohne extern angelegtes Vakuum in der Lage sein, den Knochenzement bereitzustellen. Die Vorrichtung soll mit möglichst wenigen Arbeitsschritte bedient werden können, um Fehlerquellen durch den Anwender zu minimieren. Es ist eine weitere Aufgabe der Erfindung, ein Verfahren bereitzustellen, mit dem Knochenzement aus zwei Ausgangskomponenten bereitgestellt werden kann, mittels dem mindestens ein Teil der bereits beschriebenen Aufgaben zumindest zum Teil gelöst wird.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

Eine erste Ausführungsform der Erfindung umfasst eine Vorrichtung zum Bereitstellen eines Knochenzementteigs aus zwei Ausgangskomponenten, umfassend
eine Mischeinheit aufweisend eine hohlzylinderförmige Kartusche mit einem Innenraum, wobei in dem Innenraum ein axial im Innenraum beweglicher Austragskolben angeordnet ist, welcher den Innenraum in einen proximalen Teil des Innenraums und einen distalen Teil des Innenraums unterteilt, wobei der proximale Teil und der distale Teil des Innenraums über ein Leitungsmittel fluidleitend miteinander verbunden sind,
wobei im proximalen Teil des Innenraums ein Knochenzementpulver als erste Ausgangskomponente lagert und wobei im distalen Teil des Innenraums ein axial im Innenraum beweglicher Förderkolben angeordnet ist,
und ein Reservoir für eine Monomerflüssigkeit als zweite Ausgangskomponente, welches über einen Einlasskanal mit dem distalen Teil des Innenraums zum Einleiten der Monomerflüssigkeit aus dem Reservoir in die Mischeinheit fluidleitend verbunden oder verbindbar ist,
wobei das Reservoir und die Mischeinheit über einen Auslasskanal, insbesondere einen zum Einlasskanal disjunkten Auslasskanal, fluidleitend verbunden oder verbindbar sind, über welchen ein Gas aus dem Innenraum in das Reservoir ausleitbar ist, insbesondere um ein Einleiten der Monomerflüssigkeit in die Mischeinheit zu verbessern, insbesondere zu erleichtern und/oder zu beschleunigen.

In einer Ausführungsform der Vorrichtung weist das Reservoir einen Reservoirbehälter, in dem mindestens eine fluidleitend geschlossene Ampulle mit einem Ampullenkörper und einem Ampullenkopf angeordnet ist und in der Ampulle die Monomerflüssigkeit lagert, und einen Hohlraum im Bereich des Ampullenkopfs auf,
wobei der Hohlraum fluidleitend mit dem Einlasskanal verbunden ist und eine Verbindung zur Ampulle umfasst, wobei der Ampullenkopf zumindest bereichsweise in der Verbindung angeordnet ist und der Reservoirbehälter zumindest abschnittsweise einen verformbaren Bereich umfasst, so dass ein Verkippen der Ampulle um einen Drehpunkt gegen die Verbindung ermöglicht ist.

In einer Ausführungsform der Vorrichtung weist der Einlasskanal einen geringeren Abstand zum Drehpunkt auf als der Auslasskanal.

In einer Ausführungsform der Vorrichtung mündet der Auslasskanal proximal zum Einlasskanal in den Innenraum.

In einer Ausführungsform der Vorrichtung ist der Einlasskanal an einem der Mischeinheit gegenüberliegendem Einlasskanalende als Trichter ausgebildet.

In einer Ausführungsform der Vorrichtung weist der Auslasskanal einen minimalen Auslasskanaldurchmesser auf, welcher mindestens einem halben minimalen Einlasskanaldurchmessers des Einlasskanals entspricht.

Erfindungsgemäss sind der Einlasskanal und der Auslasskanal jeweils zumindest zweiteilig ausgeformt, so dass die Mischeinheit und das Reservoir in einer ersten Kanalposition des Einlasskanals und des Auslasskanals fluidleitend miteinander verbunden sind und in einer zweiten Kanalposition des Einlasskanals und des Auslasskanals fluidleitend getrennt voneinander sind.

Erfindungsgemäss weist die Vorrichtung ein Verschlusselement auf, welches zumindest einen der Mischeinheit zugewandten Teil des zumindest zweiteiligen Einlasskanals und des zumindest zweiteiligen Auslasskanals fluidleitend verschließt oder verschließbar macht.

Erfindungsgemäss sind in einer Alternative die Mischeinheit und das Reservoir reversibel über einen ersten Formschluss, insbesondere im Bereich des Einlasskanals und des Auslasskanals, miteinander verbunden oder verbindbar.

Erfindungsgemäss ist in einer Alternative das Verschlusselement ein Drehventil, durch welches sich der der Mischeinheit zugewandte Teil des zweiteiligen Einlasskanals und des zweiteiligen Auslasskanals erstreckt und welches in einer ersten Drehventilposition den Einlasskanal und den Auslasskanal in der ersten Kanalposition belässt und durch ein Drehen in eine zweite Drehventilposition den Einlasskanal und den Auslasskanal in die zweite Kanalposition verbringt.

Erfindungsgemäss ist in einer Alternative das Verschlusselement nach einem Trennen des Reservoirs von der Mischeinheit durch Lösen des ersten Formschlusses in eine Verschlussposition verbringbar, um den der Mischeinheit zugewandten Teil des zweiteiligen Einlasskanals und des zweiteiligen Auslasskanals fluidleitend zu verschließen.

In einer Ausführungsform der Vorrichtung ist das Verschlusselement eine Schraube.

In einer Ausführungsform der Vorrichtung sind die Mischeinheit und das Reservoir reversibel über einen zweiten Formschluss miteinander verbunden oder verbindbar.

Eine Ausführungsform der Erfindung ist ein Verfahren zum Bereitstellen eines Knochenzementteigs aus zwei Ausgangskomponenten mittels einer Vorrichtung nach einer der vorhergehenden Ausführungsformen der Erfindung, umfassend die Schritte:
a. Fließen der Monomerflüssigkeit aus dem Reservoir durch den Einlasskanal in den distalen Teil des Innenraums unter gleichzeitigem Ausleiten eines Gases aus dem Innenraum durch den Auslasskanal in das Reservoir,
b. Fördern der Monomerflüssigkeit aus dem distalen Teil des Innenraums durch das Leitungsmittel in den proximalen Teil des Innenraums mittels eines Vortreibens des Förderkolbens in Richtung des Austragskolben.

In einer Ausführungsform des Verfahrens der Erfindung wird vor dem Fördern der Monomerflüssigkeit in Schritt b. der der Mischeinheit zugewandte Teil des zweiteiligen Einlasskanals und des zweiteiligen Auslasskanals durch das Verschlusselement fluidleitend verschlossen.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

Die Begriffe "proximal" und "distal" dienen lediglich der Bezeichnung der räumlich gegenüberliegenden Enden der Vorrichtung oder anderer Struktureinheiten der Vorrichtung und erlauben keinen Rückschluss auf die Orientierung in Bezug zu einem menschlichen Körper, beispielsweise eines Anwenders der Vorrichtung. "Distal zu ..." und "proximal zu..." oder ähnliche Formulierungen drücken entsprechend lediglich die räumliche Anordnung zweier Struktureinheiten der Vorrichtung zueinander aus.

### Ausführliche Beschreibung

Ein erster Gegenstand der Erfindung betrifft eine Vorrichtung zum Bereitstellen eines Knochenzementteigs aus zwei Ausgangskomponenten, umfassend
eine Mischeinheit aufweisend eine hohlzylinderförmige Kartusche mit einem Innenraum, wobei in dem Innenraum ein axial im Innenraum beweglicher Austragskolben angeordnet ist, welcher den Innenraum in einen proximalen Teil des Innenraums und einen distalen Teil des Innenraums unterteilt, wobei der proximale Teil und der distale Teil des Innenraums über ein Leitungsmittel fluidleitend miteinander verbunden sind,
wobei im proximalen Teil des Innenraums ein Knochenzementpulver als erste Ausgangskomponente lagert und wobei im distalen Teil des Innenraums ein axial im Innenraum beweglicher Förderkolben angeordnet ist,
und ein Reservoir für eine Monomerflüssigkeit als zweite Ausgangskomponente, welches über einen Einlasskanal mit dem distalen Teil des Innenraums zum Einleiten der Monomerflüssigkeit aus dem Reservoir in die Mischeinheit fluidleitend verbunden oder verbindbar ist,
wobei das Reservoir und die Mischeinheit über einen Auslasskanal fluidleitend verbunden oder verbindbar sind, über welchen ein Gas aus dem Innenraum in das Reservoir ausleitbar ist.

Die Vorrichtung dient einem Anmischen eines Knochenzementteigs aus einem Knochenzementpulver und einer Monomerflüssigkeit, wobei vor dem Anmischen das Knochenzementpulver in einer Mischeinheit der Vorrichtung lagert und die Monomerflüssigkeit in einem Reservoir der Vorrichtung lagerbar ist. Vorzugsweise lagert in dem Reservoir mindestens eine mit der Monomerflüssigkeit gefüllte Ampulle, vorzugsweise Glasampulle. Beispielsweise lagern in dem Reservoir eine oder zwei Ampullen, vorzugsweise eine oder zwei Glasampullen.

Die Mischeinheit dient einem Anmischen des Knochenzementteigs aus dem Knochenzementpulver und der Monomerflüssigkeit nach einem Fördern der Monomerflüssigkeit in die Mischeinheit, insbesondere nach einem Fördern der Monomerflüssigkeit in einen Innenraum der Mischeinheit.

Die Mischeinheit weist eine hohlzylinderförmige Kartusche auf. Unter einer hohlzylinderförmigen Kartusche ist ein rohrartiges Behältnis zu verstehen, welches einen Innenraum und eine den Innenraum umgebende Kartuschenwand aufweist. Der Querschnitt der Kartusche kann beliebige Formen annehmen. Aufgrund der einfachen Fertigung und der anwendungssichereren Verwendung der Vorrichtung ist der Querschnitt, und bevorzugt auch der Querschnitt des Innenraums, kreisförmig ausgestaltet. Dies erlaubt eine gute Handhabbarkeit für den Anwender und reduziert durch eine Abwesenheit von Kanten ein Risiko einer Verkeilung beweglicher Teile innerhalb der Vorrichtung. Erfindungsgemäß kann die Kartusche aus unterschiedlichsten Materialien oder Materialkombinationen bestehen. Beispiele kann die Vorrichtung aus einem Polymer bestehen. Bevorzugt handelt es sich bei dem Polymer um ein transparentes Polymer, da der Anwender auf diese Weise eine ordnungsgemäße Funktion der Vorrichtung während einer Verwendung optisch kontrollieren kann.

In dem Innenraum der Kartuschen ist ein axial im Innenraum beweglicher Austragskolben angeordnet, welcher den Innenraum in einen proximalen Teil des Innenraums und einen distalen Teil des Innenraums unterteilt. Im proximalen Teil des Innenraums, also proximal zum Austragskolben, lagert das Knochenzementpulver. Vorzugsweise ist der Austragskolben so ausgestattet und wirkt so mit der Kartuschenwand zusammen, dass das Knochenzementpulver im Wesentlichen nicht in den distalen Teil des Innenraums gelangen kann.

Der Austragskolben dient weiterhin dem Austragen des bereitgestellten Knochenzementteigs aus der Mischeinheit. Dazu kann der Austragskolben aus seiner ursprünglichen Position in Richtung einer Austragsöffnung der Mischeinheit verbracht werden. Die Austragsöffnung befindet sich bevorzugt an einer dem Austragskolben axial gegenüberliegenden Seite des Knochenzementpulvers und somit proximal zum Austragskolben. Um ein Gas aus der Mischeinheit, insbesondere dem proximalen Teil des Innenraums, zu entfernen, insbesondere vor dem Ausbilden des Knochenzementteigs zu entfernen, ist es bevorzugt, dass die Austragsöffnung gasdurchlässig ausgestaltet ist. Beispielsweise kann die Austragsöffnung mit einem gasleitenden Verschluss, wie beispielsweise einem Stopfen, verschlossen sein, welcher zum Austragen des angemischten Knochenzementteigs aus der Austragöffnung entfernbar ist.

Die Mischeinheit weist einen axial im Innenraum beweglichen Förderkolben auf. Der Förderkolben ist im distalen Teil des Innenraums, also distal zum Austragskolben, angeordnet. Der Förderkolben verschließt die Mischeinheit an einem distalen Kartuschenende, so dass die aus dem Reservoir in den distalen Teil des Innenraums geförderte Monomerflüssigkeit nicht aus der Kartusche ausfließen kann. In einer Ausgangsposition der Vorrichtung ist der Förderkolben so im Innenraum angeordnet, dass nach dem Fördern der Monomerflüssigkeit diese zwischen Austragskolben und Förderkolben im distalen Teil des Innenraums lagert.

Durch ein Vortreiben des Förderkolbens in Richtung des Austragskolbens, also ein Vortreiben in proximaler Richtung, kann die im distalen Teil des Innenraums gelagerte Monomerflüssigkeit durch ein Leitungsmittel in den proximalen Teil des Innenraums in das Knochenzementpulver gefördert werden, welches den proximalen Teil und den distalen Teil des Innenraums fluidleitend miteinander verbindet. Fluidleitend bedeutet, dass der distale Teil und der proximale Teil des Innenraums für Flüssigkeiten, insbesondere die Monomerflüssigkeit, und für Gase durchlässig verbunden sind. Um zu verhindern, dass Knochenzementpulver aus dem proximalen Teil in den distalen Teil des Innenraums gelangen kann, ist das Leitungsmittel vorzugsweise mit einem Filtermittel, insbesondere einer Porenscheibe, beispielsweise aus gesinterten Polypropylenpartikeln, aus gesinterten oder verpressten Polyethylenfasern, aus Cellulosefilz oder aus Pappe, ausgestattet, welche das Leitungsmittel für Feststoffe undurchlässig ausgestaltet. In einer Variante der Vorrichtung ist in dem Austragskolben und/oder zwischen dem Austragskolben und der Kartuschenwand zumindest ein Durchlass als Leitungsmittel vorgesehen, durch die der distale Teil und der proximale Teil des Innenraums fluidleitend miteinander verbunden sind. Dabei kann in oder an einem oder beiden Enden des zumindest einen Durchlasses ein für das Knochenzementpulver undurchlässiger und für die Monomerflüssigkeit und Gase durchlässiger Filter, beispielsweise eine Porenscheibe, beispielsweise aus gesinterten Polypropylenpartikeln, aus gesinterten oder verpressten Polyethylenfasern, aus Cellulosefilz oder aus Pappe, angeordnet sein. In einer weiteren Variante der Vorrichtung ist das Leitungsmittel eine oder mehrere Leitungen, die außen an der Kartusche oder in der Kartuschenwand angeordnet ist oder sind und den distalen Teil und den proximalen Teil des Innenraums verbindet oder verbinden. Der Austragskolben wird in dieser Variante umgangen.

Eine Variante der Vorrichtung ist ausgestaltet, dass ein fortgeführtes Vortreiben des Förderkolbens in Richtung des Austragskolbens nach erfolgtem Fördern der Monomerflüssigkeit vom distalen Teil des Innenraums in den proximalen Teil des Innenraums, ein Vortreiben des Austragskolbens in Richtung der Austragsöffnung der Vorrichtung bewirkt. Auf diese Weise kann der durch Mischung von Knochenzementpulver und Monomerflüssigkeit bereitgestellte Knochenzementteig durch die Austragsöffnung aus der Vorrichtung ausgetragen werden. Hiermit wird auf einfache Weise erreicht, dass der Knochenzementteig mit dem gleichen Antrieb aus der Kartusche auszutreiben ist, der auch zum Fördern der Monomerflüssigkeit verwendet wird, nämlich mit dem unidirektional angetriebenem Förderkolben.

Um ein ungewolltes Vortreiben des Austragskolbens in Richtung der Austragsöffnung zu verhindern, kann am Austragskolben ein Rastmittel angeordnet sein, so dass der Austragskolben mit der Kartusche, insbesondere mit der Kartuschenwand, rasten kann, wobei diese Rastung durch die beim Fördern der Monomerflüssigkeit in den proximalen Teil des Innenraums aufzuwendenden Druck nicht zu lösen ist, aber durch einen unmittelbaren Druck des Förderkolbens auf den Austragskolben wirkenden Druck lösbar ist.

Durch das Rastmittel wird erreicht, dass zunächst die Monomerflüssigkeit in das Knochenzementpulver, gepresst werden kann, wobei der Austragskolben dabei relativ zur Kartusche und zum Innenraum seine ursprüngliche Position hält. Erst nachdem die Monomerflüssigkeit weitgehend in das Knochenzementpulver gepresst wurde, und somit der Knochenzementteig im proximalen Teil des Innenraums der Kartusche vorliegt, kann anschließend der Knochenzementteig mit dem Austragskolben aus dem proximalen Teil der Kartusche gedrückt werden. Die Kraft zur Lösung der Rastung ist also größer als die zum Fördern der Monomerflüssigkeit über das Leitungsmittel in den proximalen Teil des Innenraums notwendige Kraft.

Das Reservoir dient dem Bereitstellen der Monomerflüssigkeit, bevor diese durch Mischen mit dem Knochenzementpulver in der Mischeinheit den Knochenzementteig bereitstellt. Vorzugsweise lagert die Monomerflüssigkeit in dem Reservoir, bis ein Anwender der Vorrichtung den Knochenzement bereitstellen möchte.

Um die Monomerflüssigkeit aus dem Reservoir in den distalen Teil des Innenraums der Mischeinheit zu fördern, sind das Reservoir und der distale Teil des Innenraums der Mischeinheit fluidleitend über einen Einlasskanal miteinander verbunden oder verbindbar. Der Einlasskanal weist dazu einen Einlasskanaldurchmesser auf, welcher ein möglichst schnelles Fördern der Monomerflüssigkeit aus dem Reservoir in die Mischeinheit erlaubt. Beispielsweise liegt der Einlasskanaldurchmesser, insbesondere ein minimaler Einlasskanaldurchmesser, in einem Bereich von 1 mm bis 4 mm.

Um das Fördern der Monomerflüssigkeit aus dem Reservoir in den distalen Teil des Innenraums zu verbessern, insbesondere zu beschleunigen, sind das Reservoir und der distale Teil des Innenraums, zusätzlich zu dem Einlasskanal, über einen, insbesondere zum Einlasskanal disjunkten, Auslasskanal fluidleitend miteinander verbunden oder verbindbar. Sind das Reservoir und der distale Teil des Innenraums fluidleitend über den Einlasskanal und den Auslasskanal miteinander verbunden, erlaubt dies ein verbessertes, insbesondere ein beschleunigtes, Fördern der Monomerflüssigkeit aus dem Reservoir über den Einlasskanal in den distalen Teil des Innenraums der Mischeinheit, während gleichzeitig ein Gas aus dem distalen Teil des Innenraums, welches durch die in den distalen Teil des Innenraums eintretende Monomerflüssigkeit aus dem distalen Teil des Innenraums verdrängt wird, über den Auslasskanal in das Reservoir ausgeleitet werden kann. Der Einlasskanal und der Auslasskanal sorgen somit synergistisch für einen verbesserten Stoffaustausch zwischen Reservoir und Mischeinheit.

Ein Fördern der Monomerflüssigkeit aus dem Reservoir über den Einlasskanal in die Mischeinheit kann beispielsweise über die Schwerkraft, über einen Unterdruck in der Mischeinheit, insbesondere im Innenraum der Kartusche, oder einer Kombination davon ausgelöst werden, wobei ein Fördern mittels der Schwerkraft bevorzugt ist. Insbesondere beim Fördern mittels Schwerkraft verbessert der Auslasskanal das Einleiten der Monomerflüssigkeit in die Mischeinheit, da das aus dem Innenraum verdrängte Gas in das Reservoir ableitbar ist. Das Reservoir kann aus unterschiedlichsten Materialien oder Materialkombinationen bestehen. Beispiele kann das Reservoir aus einem Polymer bestehen. Bevorzugt handelt es sich bei dem Polymer um ein transparentes Polymer, da der Anwender auf diese Weise eine ordnungsgemäße Funktion des Reservoirs, insbesondere ein Ausfließen der Monomerflüssigkeit aus dem Reservoir, während einer Verwendung optisch kontrollieren kann.

Das Reservoir kann unterschiedlich ausgestaltet sein, um die Monomerflüssigkeit bereitzustellen. Beispielsweise kann die Monomerflüssigkeit einen Reservoirinnenraum aufweisen, in welchem die Monomerflüssigkeit frei fließend lagert.

Vorzugsweise ist die Monomerflüssigkeit innerhalb des Reservoirs in einem oder mehreren separaten Behältnissen gelagert, was die Handhabung und Befüllung der Vorrichtung, insbesondere des Reservoirs, und die sterile Bereitstellung der Monomerflüssigkeit erleichtert. Beispielsweise wird die Monomerflüssigkeit im Reservoir in einem Behälter in Form eines Beutels bereitgestellt. Unter einem Beutel wird eine nicht-starre, weitestgehend flexible Lagermöglichkeit verstanden, welche die Monomerflüssigkeit hermetisch dicht und steril lagern kann und mittels Einwirkung eines Öffnungsmittels, beispielsweise durch Aufstechen, Aufschneiden oder Aufreißen, zu öffnen ist. Die Beutel können beispielsweise aus einer Mehrschichtverbundfolie, vorzugsweise aufweisend eine EVOH-Sperrschicht gefertigt sein. Optional können die Beutel eine Metallbeschichtung, insbesondere eine Aluminiumbeschichtung, aufweisen.

Vorzugsweise lagert in dem Reservoir mindestens eine die Monomerflüssigkeit enthaltende Ampulle, vorzugsweise Glasampulle. Beispielsweise lagern in dem Reservoir zwei die Monomerflüssigkeit enthaltende Ampullen, vorzugsweise Glasampullen. Ampullen, insbesondere Glasampullen, sind aufgrund einer guten Sterilisierbarkeit und der leichten und zuverlässigen Öffenbarkeit durch manuelle Krafteinwirkung bevorzugt.

Eine Ausführungsform der Vorrichtung ist dadurch gekennzeichnet, dass das Reservoir einen Reservoirbehälter, in dem mindestens eine fluidleitend geschlossene Ampulle mit einem Ampullenkörper und einem Ampullenkopf angeordnet ist und in der Ampulle die Monomerflüssigkeit lagert, und einen Hohlraum im Bereich des Ampullenkopfs aufweist, wobei der Hohlraum fluidleitend mit dem Einlasskanal verbunden ist und eine Verbindung zur Ampulle umfasst, wobei der Ampullenkopf zumindest bereichsweise in der Verbindung angeordnet ist und der Reservoirbehälter zumindest abschnittsweise einen verformbaren Bereich umfasst, so dass ein Verkippen der Ampulle um einen Drehpunkt gegen die Verbindung ermöglicht ist.

In dieser Ausführungsform weist das Reservoir einen Reservoirbehälter zur Aufnahme einer oder mehrerer, vorzugsweise zweier, mit der Monomerflüssigkeit befüllten fluidleitend geschlossenen Ampulle beziehungsweise Ampullen, insbesondere einer Glasampulle beziehungsweise Glasampullen, mit einem Ampullenkopf und einem Ampullenkörper auf. Der Reservoirbehälter umschließt die mindestens eine Ampulle, insbesondere zumindest den Ampullenkörper, so dass die Ampulle sicher im Reservoir bis zu ihrer Verwendung lagerbar ist. Der Reservoirbehälter kann beispielsweise in der Form eines Hohlzylinders vorliegen, in den die mindestens eine Ampulle eingeschoben ist, wobei zur verbesserten Transportfähigkeit der Vorrichtung der Reservoirbehälter so ausgeformt ist, beispielsweise indem der Reservoirbehälter einen Deckel aufweist, dass die Ampulle nicht unbeabsichtigt aus der Vorrichtung, insbesondere dem Reservoir, austreten kann. Vorzugsweise ist der Reservoirbehälter so ausgeformt, dass zwei Ampullen, insbesondere zwei Ampullen nebeneinander, vorzugsweise mit im Wesentlichen parallelen Längsachsen, in dem Reservoir gelagert werden können.

Weiterhin dient das Reservoir dem fluidleitenden Öffnen der mindestens einen Ampulle. Dazu weist das Reservoir einen Hohlraum auf, welcher über eine Verbindung mit der im Reservoirbehälter angeordneten Ampulle verbunden ist. Die Ampulle ist derart im Reservoir gelagert, dass der Ampullenkopf in Richtung des Hohlraums zeigt, während der Ampullenkörper zumindest anteilig, vorzugsweise vollständig, im Reservoirbehälter angeordnet ist. Zwischen Hohlraum und Reservoirbehälter erstreckt sich die Verbindung, und zwar derart, dass der Ampullenkopf zumindest bereichsweise in der Verbindung angeordnet ist. Dazu weist die Verbindung einen Verbindungsdurchmesser auf, welcher ein zumindest abschnittsweises Einschieben des Ampullenkopfs in die Verbindung erlaubt. In einer Ausgestaltungsform weist die Verbindung einen Verbindungsdurchmesser auf, welcher ein vollständiges Einschieben des Ampullenkopfs in die Verbindung erlaubt. Bevorzugt ist der Verbindungsdurchmesser kleiner als der Durchmesser des Ampullenkörpers, so dass dieser nicht in die Verbindung einschiebbar ist. Beispielsweise ist die Verbindung als Ring oder Hohlzylinder ausgestaltet und der Ampullenkopf ist zumindest bereichsweise von diesem Ring oder Hohlzylinder umgeben. Die Verbindung weist eine strukturelle Integrität auf, welche eine strukturelle Integrität der Ampulle übersteigt, so dass bei einem Pressen der Ampulle gegen die Verbindung die Ampulle zerbrechen kann. Um die Ampulle, oder bei der Anwesenheit von zwei oder mehr Ampullen alle Ampullen, zu öffnen, weist der Reservoirbehälter zumindest abschnittsweise, insbesondere angrenzend an einen Übergang des Ampullenkopfs zum Ampullenkörper der Ampulle, einen verformbaren Bereich auf. In einer Ausgestaltungsform ist der Reservoirbehälter vollständig verformbar. Der verformbare Bereich erlaubt ein Verkippen der Ampulle um einen Drehpunkt gegen die Verbindung. Dabei ist der Verbindungsdurchmesser so auf den Ampullenkopf abgestimmt, dass beim Verkippen zumindest das dem Ampullenkopf abgewandte Ampullenkörperende um den Drehpunkt verkippt wird, während zumindest das dem Ampullenkörper abgewandte Ampullenkopfende innerhalb der Verbindung verbleibt, so dass die Ampulle durch ein zumindest teilweises Zerbersten der Ampulle, insbesondere im Bereich eines Ampullenhalses zwischen Ampullenkopf und Ampullenkörper, fluidleitend geöffnet wird. Die Verbindung dient dabei hauptsächlich der Fixierung des Ampullenkopfs gegen eine Kippbewegung der Ampulle um den Drehpunkt. Beispielsweise ist der Verbindungsdurchmesser um nicht mehr als 10 % größer als der Durchmesser des Ampullenkopfs, so dass bereits ein relativ geringfügiges Verkippen der Ampulle zu deren fluidleitenden Öffnung führt.

Nach einem fluidleitenden Öffnen der zumindest einen Ampulle kann die Monomerflüssigkeit aus der Ampulle in den Hohlraum fließen. Der Hohlraum ist über den Einlasskanal fluidleitend mit der Mischeinheit, insbesondere mit dem distalen Teil des Innenraums der Mischeinheit, verbunden. Ein Fördern der Monomerflüssigkeit aus dem Hohlraum über den Einlasskanal in die Mischeinheit kann beispielsweise über die Schwerkraft, über einen Unterdruck in der Mischeinheit, insbesondere im Innenraum der Kartusche, oder einer Kombination davon ausgelöst werden, wobei ein Fördern mittels der Schwerkraft bevorzugt ist. Insbesondere beim Fördern mittels Schwerkraft verbessert der Auslasskanal das Einleiten der Monomerflüssigkeit in die Mischeinheit.

Eine Ausführungsform der Vorrichtung ist dadurch gekennzeichnet, dass der Einlasskanal einen geringeren Abstand zum Drehpunkt als der Auslasskanal aufweist. Insbesondere weist ein der Mischeinheit gegenüberliegendes, dem Reservoir zugewandtes Einlasskanalende einen geringeren Abstand, insbesondere eine geringere räumliche Entfernung, zum Drehpunkt auf als ein der Mischeinheit gegenüberliegendes, dem Reservoir zugewandtes Auslasskanalende.

Die mindestens eine Ampulle wird beim Verkippen um den Drehpunkt gegen die Verbindung in der Umgebung des Drehpunkts, insbesondere im Bereich des Ampullenhalses, fluidleitend geöffnet, so dass die Monomerflüssigkeit aus der mindestens einen Ampulle in den Hohlraum ausfließen kann. Ist der Einlasskanal, insbesondere das der Mischeinheit gegenüberliegende Einlasskanalende näher am Drehpunkt angeordnet als der Auslasskanal, insbesondere das der Mischeinheit gegenüberliegende Auslasskanalende, so wird die Monomerflüssigkeit, bei im Wesentlichen senkrechter räumlicher Ausrichtung der Vorrichtung und ohne weiteres Zutun eines Anwenders der Vorrichtung, im Wesentlichen vollständig durch den Einlasskanal in die Mischeinheit, insbesondere den distalen Teil des Innenraums der Mischeinheit, einfließen, während gleichzeitig der Auslasskanal im Wesentlichen frei von der Mischeinheit verbleibt und so ein verbessertes Ausleiten des aus dem Innenraum verdrängten Gases zulässt. Diese Anordnung von Einlasskanal und Auslasskanal zueinander verbessert somit den Stoffaustausch zwischen Mischeinheit und Reservoir.

Der Einlasskanal und der Auslasskanal können auf gleicher räumlicher Höhe entlang einer Längsachse der Vorrichtung, insbesondere der Mischeinheit, in den Innenraum, insbesondere den distalen Teil des Innenraums, münden. In dieser Ausführungsform münden der Einlasskanal und der Auslasskanal im weitesten Sinne "nebeneinander" in den distalen Teil des Innenraums der Mischeinheit.

Eine Ausführungsform der Vorrichtung ist dadurch gekennzeichnet, dass der Auslasskanal proximal zum Einlasskanal in den Innenraum, insbesondere in den distalen Teil des Innenraums, mündet. Der Auslasskanal, insbesondere ein der Mischeinheit zugewandtes Auslasskanalende, liegt somit näher am Austragskolben als der Einlasskanal, insbesondere ein der Mischeinheit zugewandtes Einlasskanalende. Somit mündet der Einlasskanal, insbesondere das der Mischeinheit zugewandte Einlasskanalende, näher am Förderkolben als der Auslasskanal, insbesondere das der Mischeinheit zugewandte Auslasskanalende. Dadurch reduziert sich die Gefahr eines Ausfließens der Monomerflüssigkeit aus dem Innenraum durch den Auslasskanal direkt nach dem Einfließen in den Innenraum durch den Einlasskanal, insbesondere falls, wie aufgrund einer möglichst kompakten Bauweise der Vorrichtung bevorzugt, der Einlasskanal und der Auslasskanal nahe beieinander in den distalen Teil des Innenraums münden. Vorzugsweise münden der Auslasskanal und der Einlasskanal nicht weiter als 1 cm entfernt zueinander in den Innenraum.

Um sicherzustellen, dass die Monomerflüssigkeit aus dem Reservoir, vorzugsweise aus der mindestens einen im Reservoir gelagerten, fluidleitend geöffneten Ampulle, im Wesentlichen vollständig über den Einlasskanal, und nicht über den Auslasskanal, in den distalen Teil des Innenraums gefördert wird, ist eine Ausführungsform der Vorrichtung dadurch gekennzeichnet, dass der Einlasskanal an dem der Mischeinheit gegenüberliegenden, dem Reservoir zugewandten Einlasskanalende als Trichter ausgebildet ist. Die Ausgestaltung als Trichter erleichtert das Einfließen der Monomerflüssigkeit in den Einlasskanal. Insbesondere beim Lagern der Monomerflüssigkeit in einer Ampulle, insbesondere einer Glasampulle, kann die Ausformung als Trichter vorteilhaft sein, da die Monomerflüssigkeit aus der mindestens einen fluidleitend geöffneten Ampulle möglicherweise unregelmäßig, beispielsweise stoßweise, ausfließen kann. Die Ausformung als Trichter verbessert insbesondere bei unregelmäßigem Fließen der Monomerflüssigkeit die Aufnahme derselben in den Einlasskanal. Der Trichter kann beispielsweise einen Durchmesser in einem Bereich von 1 cm bis 4 cm einnehmen. Die Querschnittsfläche des Trichters kann beispielsweise rund, eckig oder elliptisch ausgestaltet sein.

Die Ausformung als Trichter kann unterschiedliche Anteile der Gesamtlänge des Einlasskanals einnehmen. Beispielsweise kann der Einlasskanal über 10 % bis 95 %, vorzugsweise über 30 % bis 90 %, weiter bevorzugt über 50 % bis 90 %, der Gesamtlänge des Einlasskanals als Trichter ausgestaltet sein.

Der Auslasskanal und der Einlasskanal können unterschiedliche Durchmesser zueinander aufweisen.

Eine Ausführungsform der Vorrichtung ist dadurch gekennzeichnet, dass der Auslasskanal einen minimalen Auslasskanaldurchmesser aufweist, welcher mindestens dem halben minimalen Einlasskanaldurchmesser des Einlasskanals entspricht. Der minimale Einlasskanaldurchmesser ist somit bevorzugt maximal doppelt so groß wie der minimale Auslasskanaldurchmesser.

Dies stellt sicher, dass das Gas aus dem Innenraum genügend schnell über den Auslasskanal in das Reservoir abgeführt werden kann, um das Fördern der Monomerflüssigkeit aus dem Reservoir in den Innenraum nicht zu verlangsamen.

In einer bevorzugten Ausgestaltungsform sind der minimale Auslasskanaldurchmesser und der minimale Einlasskanaldurchmesser im Wesentlichen gleich groß.

Der Einlasskanal und der Auslasskanal sind zumindest zweiteilig, bevorzugt zwei- oder dreiteilig, weiter bevorzugt zweiteilig, ausgeformt sind, so dass die Mischeinheit, insbesondere der distale Teil des Innenraums der Mischeinheit, und das Reservoir, vorzugsweise der Hohlraum des Reservoirs, in einer ersten Kanalposition des Einlasskanals und des Auslasskanals fluidleitend miteinander verbunden sind und in einer zweiten Kanalposition des Einlasskanals und des Auslasskanals fluidleitend getrennt voneinander sind. Vorzugsweise sind lassen sich der Einlasskanal und der Auslasskanal reversibel in die erste Kanalposition und die zweite Kanalposition verbringen. Ein einteiliger Kanal kann nicht reversibel zwei Kanalpositionen einnehmen.

Dabei sind an der Ausformung des Einlasskanals und des Auslasskanals zumindest zwei Bauteile, bevorzugt zwei oder drei Bauteile, weiter bevorzugt zwei Bauteile, beteiligt und der Einlasskanal und der Auslasskanal erstrecken sich durch diese Bauteile. Beispielsweise ist der Einlasskanal durch ein fluidleitendes Verbinden von zwei disjunkten Schläuchen ausgebildet.

Vorzugsweise verläuft der der Mischeinheit zugwandte Teil der Kanäle in einem der Bauteile und der dem Reservoir zugewandte Teil der Kanäle in einem anderen Bauteil.

Vorzugsweise werden der Einlasskanal und der Auslasskanal durch dieselben Bauteile ausgebildet.

Durch die zumindest zweiteilige Ausgestaltung von Einlasskanal und Auslasskanal können beide Kanäle, vorzugsweise reversibel, in einer ersten Kanalposition eine fluidleitende Verbindung zwischen Reservoir und Mischeinheit herstellen und in einer zweiten Kanalposition das Reservoir und die Mischeinheit fluidleitend trennen. Beispielsweise ist der Einlasskanal durch ein fluidleitendes Verbinden von zwei vormals disjunkten Schläuchen ausgebildet, wobei die fluidleitend miteinander verbundenen Schläuche die erste Kanalposition darstellen und die zwei getrennten Schläuche die zweite Kanalposition darstellen.

Durch die zumindest zweiteilige Ausgestaltung des Einlasskanals und des Auslasskanals kann ein Anwender der Vorrichtung somit den Stoffaustausch zwischen der Mischeinheit und dem Reservoir kontrollieren.

Vorzugsweise können der Einlasskanal und der Auslasskanal gleichzeitig in die erste oder zweite Kanalposition verbracht werden.

Die Vorrichtung weist ein Verschlusselement auf, welches zumindest einen der Mischeinheit zugewandten, also an die Mischeinheit angrenzenden, Teil des zumindest zweiteiligen Einlasskanals und des einen der Mischeinheit zugewandten, also an die Mischeinheit angrenzenden, Teil des zumindest zweiteiligen Auslasskanals fluidleitend verschließt oder verschließbar macht, vorzugsweise reversibel fluidleitend verschließt oder verschließbar macht. Beispielsweise umfasst die Vorrichtung einen Stopfen, welcher in das der Mischeinheit zugewandte Einlasskanalende und Auslasskanalende reversibel einführbar ist, um die Mischeinheit und das Reservoir fluidleitend zu trennen.

Das Verschlusselement vereinfacht ein im Wesentlichen vollständiges Fördern der Monomerflüssigkeit aus dem distalen Teil des Innenraums durch das Leitungsmittel in den proximalen Teil des Innenraums. Ohne das Verschlusselement könnten die Monomerflüssigkeit, oder Anteile davon, beim Fördern derselben in den proximalen Teil des Innenraums durch den Einlasskanal und/oder den Auslasskanal wieder aus der Mischeinheit ausgeleitet werden.

Eine Ausführungsform der Vorrichtung ist dadurch gekennzeichnet, dass die Mischeinheit und das Reservoir reversibel über einen ersten Formschluss miteinander verbunden oder verbindbar sind. Somit sind die Mischeinheit und das Reservoir reversibel trennbar zueinander ausgestaltet, was die Verwendung der Vorrichtung für einen Anwender vereinfacht. Dies erlaubt ein einfaches Abtrennen des Reservoirs, welches nach dem Fördern der Monomerflüssigkeit in die Mischeinheit, insbesondere in den distalen Teil des Innenraums der Mischeinheit, nicht mehr benötigt wird, wodurch das Bereitstellen, und vorzugsweise das Austragen, des Knochenzementteigs mittels der Mischeinheit, insbesondere durch eine Verbesserung der Handlichkeit, vereinfacht wird.

Vorzugsweise sind an der Ausbildung des ersten Formschlusses dieselben Bauteile beteiligt, welche den zumindest zweiteiligen Einlasskanal und den zumindest zweiteiligen Auslasskanal ausbilden.

In einer Ausgestaltungsform befinden sich der Einlasskanal und der Auslasskanal in der ersten Kanalposition, wenn der erste Formschluss ausgebildet ist und in der zweiten Kanalposition, wenn der erste Formschluss gelöst ist.

Das Verschlusselement kann unterschiedlich ausgeformt sein, um die Mischeinheit und das Reservoir fluidleitend zu trennen.

Eine Ausführungsform der Vorrichtung ist dadurch gekennzeichnet, dass das Verschlusselement ein Drehventil ist, durch welches sich der der Mischeinheit zugewandte Teil des zweiteiligen Einlasskanals und des zweiteiligen Auslasskanals, also der an die Mischeinheit angrenzende Teil des zweiteiligen Einlasskanals und des zweiteiligen Auslasskanals, erstreckt und welches in einer ersten Drehventilposition den Einlasskanal und den Auslasskanal in der ersten Kanalposition belässt und durch ein Drehen in ein zweite Drehventilposition den Einlasskanal und den Auslasskanal in die zweite Kanalposition verbringt. Der der Mischeinheit abgewandte, dem Reservoir zugewandte Teil des zumindest zweiteiligen Einlasskanals und zweiteiligen Auslasskanals erstreckt sich jeweils nicht durch das Drehventil. Das Drehventil bildet somit dasjenige Bauteil des zumindest zweiteiligen Einlasskanals und Auslasskanals aus, welches die der Mischeinheit zugewandten Teile der beiden Kanäle ausformt. Ist das Drehventil in der ersten Drehventilposition, befinden sich der Einlasskanal und der Auslasskanal in der ersten Kanalposition, wodurch die Mischeinheit, insbesondere der distale Teil des Innenraums der Mischeinheit, und das Reservoir fluidleitend miteinander verbunden sind. Ist das Drehventil in der zweiten Drehventilposition, befinden sich der Einlasskanal und der Auslasskanal in der zweiten Kanalposition, wodurch die Mischeinheit, insbesondere der distale Teil des Innenraums der Mischeinheit, und das Reservoir fluidleitend getrennt voneinander sind. Durch das Drehen des Drehventils in die zweite Drehventilposition, und somit das Verbringen des Einlasskanals und des Auslasskanals in die zweite Kanalposition, werden die zwei Teile des Einlasskanals und die zwei Teile des Auslasskanals derart räumlich zueinander verschoben, dass der Stoffaustausch zwischen den jeweiligen Teilen des Einlasskanals und des Auslasskanals unterbunden ist. Durch das Drehen des Drehventils in die erste Drehventilposition, und somit das Verbringen des Einlasskanals und des Auslasskanals in die erste Kanalposition, werden die zwei Teile des Einlasskanals und die zwei Teile des Auslasskanals derart zueinander angeordnet, dass ein Stoffaustausch zwischen den entsprechenden Teilen des Einlasskanals und des Auslasskanals ermöglicht ist.

Eine Ausführungsform der Vorrichtung ist dadurch gekennzeichnet, dass das Verschlusselement nach einem Trennen des Reservoirs von der Mischeinheit durch Lösen des ersten Formschlusses in eine Verschlussposition verbringbar ist, um den der Mischeinheit zugewandten Teil des zweiteiligen Einlasskanals und des zweiteiligen Auslasskanals fluidleitend zu verschließen. In dieser Ausführungsform werden durch Lösen des ersten Formschlusses die Teile des zumindest zweiteiligen Einlasskanals und des zweiteiligen Auslasskanals fluidleitend getrennt, wobei vorzugsweise jeweils der dem Reservoir zugewandte, also an das Reservoir angrenzende, Teil des Einlasskanals und des Auslasskanals zusammen mit dem Reservoir durch Lösen des ersten Formschlusses von der Mischeinheit entfernt wird und die an die Mischeinheit angrenzenden Teile des Einlasskanals und des Auslasskanals an der Mischeinheit, beispielsweise als Durchführungen in der Kartuschenwand im Bereich des distalen Teil des Innenraums, verbleiben. Durch Lösen des ersten Formschlusses werden in dieser Ausführungsform der Einlasskanal und der Auslasskanal aus der ersten Kanalposition in die zweite Kanalposition verbracht. Das Verschlusselement dient dazu, nach dem Entfernen des Reservoirs, die an der Mischeinheit verbleibenden Teile des Einlasskanals und des Auslasskanals, vorzugsweise von außerhalb der Mischeinheit, zu verschließen. Dazu wird das Verschlusselement in die Verschlussposition gebracht. Beispielsweise ist das Verschlusselement ein Stopfen, welcher durch Einschieben in den an der Mischeinheit verbleibenden Teil des Einlasskanals und des Auslasskanals, vorzugsweise von außerhalb der Mischeinheit, in die Verschlussposition verbringbar ist.

Eine Ausführungsform der Vorrichtung ist dadurch gekennzeichnet, dass das Verschlusselement eine Schraube ist. Die Schraube weist vorzugsweise ein Außengewinde auf, welches mit einem Innengewinde, welches vorzugsweise außerhalb der Mischeinheit angebracht ist, derart zusammenwirkt, dass, nach dem Lösen des ersten Formschlusses und dem Entfernen des dem Reservoir zugewandten Teils des Einlasskanals und des Auslasskanals, ein Eindrehen der Schraube in Richtung der Kartuschenwand ein fluidleitendes Verschließen des an der Mischeinheit verbliebenen Teils des Einlasskanals und des Auslasskanals, vorzugsweise durch eine Schraubenspritze, bewirkt wird.

Vorzugsweise weist die Schraube Flügel auf, um einem Anwender der Vorrichtung das Einschrauben in die Verschlussposition zu erleichtern.

Der erste Formschluss kann die einzige mechanische Verbindung zwischen der Mischeinheit und dem Reservoir darstellen.

Eine Ausführungsform der Vorrichtung ist dadurch gekennzeichnet, dass die Mischeinheit und das Reservoir reversibel, zusätzlich zu dem ersten Formschluss, über einen zweiten Formschluss miteinander verbunden oder verbindbar sind.

In dieser Ausführungsform sind die Mischeinheit und das Reservoir über zwei Formschlüsse miteinander verbunden.

Beim Öffnen der mindestens einen Ampulle durch ein Verkippen um den Drehpunkt gegen die Verbindung muss eine ausreichend hohe Kraft auf die Ampulle ausgeübt werden, um die strukturelle Integrität der Ampulle zu überwinden. Diese Kraft erhöht sich zusätzlich bei der Verwendung von mehr als einer Ampulle, wie vorzugsweise zwei Ampullen, falls diese, wie bevorzugt, gleichzeitig durch Verkippen um den Drehpunkt gegen die Verbindung geöffnet werden sollen. Diese Kraft wirkt auf die Kontaktstellen von Reservoir und Mischeinheit ein. Falls das Reservoir und die Mischeinheit lediglich über den ersten Formschluss verbunden wären, würde die Kraft zum Öffnen der mindestens einen Ampulle vollständig diesen ersten Formschluss einwirken, so dass es, da bevorzugt die Bauteile am ersten Formschluss beteiligt sind, welche den zumindest zweiteiligen Einlasskanals und den zumindest zweiteiligen Auslasskanal ausbilden, zu einem Abknicken oder gar Abreißens des Einlasskanals und/oder des Auslasskanals kommen könnte. Dadurch würde ein im Wesentlichen vollständiges Fördern der Monomerflüssigkeit aus dem Reservoir in die Mischeinheit, insbesondere den distalen Teil des Innenraums erschwert oder gar verhindert.

Der zweite Formschluss zwischen dem Verbindungselement und der Mischeinheit sorgt für eine Kraftverteilung der zum Öffnen der mindestens einen Ampulle benötigten Kraft auf die zwei Formschlüsse, so dass das Risiko eines Beschädigens der Vorrichtung beim Öffnen der mindestens einen Ampulle durch ein Verkippen verringert wird.

Durch die zwei Formschlüsse ist das Reservoir derart stabil mit der Mischeinheit verbunden, dass die mindestens eine Ampulle durch ein Verkippen um den Drehpunkt gegen die Verbindung geöffnet werden kann, ohne die Vorrichtung zu beschädigen und ohne zusätzliche Hilfsmittel neben der Vorrichtung zum Öffnen der mindestens einen Ampulle zu benötigen.

Der zweite Formschluss zwischen der Mischeinheit und dem Reservoir kann auf unterschiedliche Weisen realisiert werden.

In einer Ausführungsform der Vorrichtung wird der zweite Formschluss mittels einer Spange ausgebildet. Vorzugsweise ist die Spange aus zwei Spangeneinkerbungen an einer Reservoiraußenfläche, wie beispielsweise einer Außenfläche des Reservoirbehälters, des Hohlraums oder der Verbindung, sowie zwei Spangenausstülpungen an einer Mischelementaußenfläche, vorzugsweise einer Kartuschenaußenfläche, gebildet, wobei die zwei Spangenaustülpungen reversibel in die zwei Spangeneinkerbungen einschiebbar sind, um den zweiten Formschluss auszubilden. Dies erlaubt einen schnell und einfach erstell- und lösbaren zweiten Formschluss, welcher stabil ist und ein sicheres Öffnen der mindestens einen Ampulle durch Verkippen um den Drehpunkt erlaubt.

Der Drehpunkt sowie der erste Formschluss und der zweite Formschluss können räumlich auf unterschiedliche Weise zueinander angeordnet sein.

Einer Ausführungsform der Vorrichtung ist dadurch gekennzeichnet, dass der Drehpunkt, der erste Formschluss und der zweite Formschluss in einer Seitenansicht, insbesondere einer Seitenansicht der Vorrichtung, die Eckpunkte eines Dreiecks ausbilden. In dieser Ausführungsform liegen der Drehpunkt und die beiden Formschlüsse in einer gemeinsamen Ebene, sind allerdings nicht auf einer in dieser Ebene verlaufenden Geraden angeordnet. Vorzugsweise liegt die Längsachse der Kartusche innerhalb dieser Ebene oder verläuft zumindest parallel zu dieser Ebene. Die Anordnung in Form eines Dreiecks verbessert die Kraftverteilung der zum fluidleitenden Öffnen der zumindest einen Ampulle benötigten Kraft, insbesondere bei einer dazu verwendeten Verkippbewegung der Ampulle um den Drehpunkt innerhalb oder parallel zu der Ebene des Dreiecks. Weiterhin ist durch eine derartige Anordnung der Drehpunkt in dieser Ebene fixiert und verschiebt sich nicht, was ein reproduzierbares Öffnen der mindestens einen Ampulle erleichtert.

Der Drehpunkt sowie der erste Formschluss und der zweite Formschluss können unterschiedlich große Abstände zueinander aufweisen.

Eine Ausführungsform der Vorrichtung ist dadurch gekennzeichnet, dass der zweite Formschluss einen kürzeren Abstand zum ersten Formschluss als der Drehpunkt aufweist. In dieser Ausführungsform ist der Abstand zwischen Drehpunkt und erstem Formschluss somit größer als der Abstand zwischen zweitem Formschluss und erstem Formschluss. Insbesondere bei einer Anordnung von Drehpunkt und den zwei Formschlüssen in Form eines Dreiecks erlaubt dies sowohl eine verbesserte Kraftverteilung der beim Verkippen zum Öffnen der Ampulle benötigten Kraft auf die zwei Formschlüsse als auch gleichzeitig eine möglichst platzsparende Ausgestaltung der Vorrichtung. Letzteres erleichtert insbesondere die Handhabung der Vorrichtung durch einen Anwender.

Vorzugsweise bilden dabei der Drehpunkt und die zwei Formschlüsse in einer Seitenansicht die Eckpunkte eines Dreiecks, wobei der Drehpunkt und der zweite Formschluss von den drei möglichen Abständen zwischen den genannten Punkten den geringsten Wert aufweisen. Dies führt zu einer weiteren Verbesserung der Kraftverteilung auf die zwei Formschlüsse und erlaubt eine weiter platzsparende Ausgestaltung der Vorrichtung.

Eine Ausführungsform der Vorrichtung ist dadurch gekennzeichnet, dass der erste Formschluss, der zweite Formschluss und der Drehpunkt jeweils auf einer parallel zu einer Längsachse der Kartusche verlaufenden Geraden liegen, wobei die Geraden einen unterschiedlichen Geradenabstand zur Längsachse der Kartusche aufweisen. Diese Anordnung verbessert die Kraftverteilung der beim Verkippen zum Öffnen der Ampulle benötigten Kraft auf die zwei Formschlüsse und erlaubt auch gleichzeitig eine möglichst platzsparende Ausgestaltung der Vorrichtung. Letzteres erleichtert insbesondere die Handhabung der Vorrichtung durch einen Anwender.

Vorzugsweise bilden dabei der Drehpunkt und die zwei Formschlüsse die Eckpunkte eines Dreiecks, wobei das Dreieck in einer Ebene liegt, in welcher auch die Längsachse der Kartusche liegt oder zu der die Längsachse der Kartusche zumindest parallel verläuft.

Ein weiterer Gegenstand der Erfindung betrifft ein Verfahren zum Bereitstellen eines Knochenzementteigs aus zwei Ausgangskomponenten mittels einer erfindungsgemässen Vorrichtung umfassend die folgenden Schritte:
a. Fließen der Monomerflüssigkeit aus dem Reservoir durch den Einlasskanal in den distalen Teil des Innenraums unter gleichzeitigem Ausleiten eines Gases aus dem Innenraum durch den Auslasskanal in das Reservoir,
b. Fördern der Monomerflüssigkeit aus dem distalen Teil des Innenraums durch das Leitungsmittel in den proximalen Teil des Innenraums mittels eines Vortreibens des Förderkolbens in Richtung des Austragskolben.

Vorzugsweise erfolgt das Fließen der Monomerflüssigkeit in Schritt a. gemäß der Schwerkraft. Dazu wird die Vorrichtung von einem Anwender räumlich so gehalten, dass der Austragskolben räumlich über dem Förderkolben, bevorzugt senkrecht über dem Förderkolben, angeordnet ist. Beim Fließen der Monomerflüssigkeit durch den Einlasskanal in den distalen Teil des Innenraums kann sich ein temporärer Monomerflüssigkeitspegel innerhalb des Einlasskanals ausbilden, welcher sich aus einem Eintrittsvolumen der Monomerflüssigkeit in den Einlasskanal und aus einem Austrittsvolumen der Monomerflüssigkeit aus dem Einlasskanal in den distalen Teil des Innenraums ergibt. Durch die vorgenannten Ausführungsformen der Vorrichtung ist der Monomerflüssigkeitspegel vorzugsweise immer distal dem, dem Reservoir zugewandten, Austrittskanalende des Austrittskanals ausgebildet, so dass, bei senkrechter Ausrichtung der Vorrichtung mit dem proximalen Kartuschenende nach oben, im Wesentlichen keine Monomerflüssigkeit in den Austrittskanal auf Seite des Reservoirs gelangt.

Durch das Vortreiben des Förderkolbens in Richtung des Austragskolbens in Schritt b. verringert sich der räumliche Abstand zwischen den beiden Kolben, so dass, in Abhängigkeit des Volumend der im distalen Teil des Innenraums vorhanden Monomerflüssigkeit, ab einer bestimmten räumlichen Nähe die Monomerflüssigkeit aus dem distalen Teil des Innenraums durch das Leitungsmittel in den proximalen Teil des Innenraums gefördert wird.

Mit Beginn des Förderns der Monomerflüssigkeit in den proximalen Teil des Innenraums kommt es zu einem Kontakt von Monomerflüssigkeit in dem im proximalen Teil des Innenraums gelagerten Knochenzementpulver, was mit einer Ausbildung des Knochenzementteigs einhergeht.

Eine Ausführungsform des Verfahrens ist dadurch gekennzeichnet, dass vor dem Fördern der Monomerflüssigkeit in Schritt b. der der Mischeinheit zugewandte Teil des zweiteiligen Einlasskanals und des zweiteiligen Auslasskanals durch das Verschlusselement fluidleitend verschlossen wird.

Dies erleichtert ein im Wesentlichen vollständiges Fördern der Monomerflüssigkeit aus dem distalen Teil des Innenraums durch das Leitungsmittel in den proximalen Teil des Innenraums, ohne dass wesentliche Anteile der Monomerflüssigkeit durch das Vortreiben des Förderkolbens aus dem distalen Teil des Innenraums durch den Einlasskanal und/oder den Auslasskanal aus der Mischeinheit herausgeleitet werden. Weiterhin muss ein Anwender die Vorrichtung nicht räumlich genau so ausrichten, dass dieses Herausleiten im Wesentlichen unterbleibt.

Der Förderkolben kann auf unterschiedliche Weisen in Richtung des Austragskolben vorgetrieben werden. Beispielsweise kann ein Anwender der Vorrichtung den Förderkolben manuell, insbesondere über Krafteinwirkung auf eine Stange oder Achse, vortreiben. In einer weiteren Ausführungsform bilden die Kartusche und der Förderkolben zusammen ein Gewinde aus, über welche der Förderkolben in Richtung des Austragskolben in die Kartusche hineingeschraubt werden kann. Dabei weist vorzugsweise die Kartusche ein Innengewinde und der Förderkolben ein Außengewinde auf, welche form- und/oder kraftschlüssig zusammenwirken, um das Vortreiben des Förderkolbens zu ermöglichen.

In einer weiteren Ausführungsform des Verfahrens erfolgt das Vortreiben des Förderkolbens unter Einsatz eines mechanischen Hilfsmittels.

Eine Ausführungsform des Verfahrens ist dadurch gekennzeichnet, dass zum Vortreiben des Förderkolbens die Vorrichtung in eine Austragseinrichtung, insbesondere eine Austragspistole für Knochenzementteige, eingesetzt wird. Austragspistolen für Knochenzementteige sind dem Fachmann bekannt.

Mit dem Fördern der Monomerflüssigkeit aus dem hinteren Teil in den vorderen Teil des Innenraums beginnt die Ausbildung des Knochenzementteigs aus den beiden Ausgangskomponenten. Vorzugsweise erfolgt dies unter einer möglichst gleichmäßen Durchmischung der beiden Ausgangskomponenten, um einen möglichst homogenen Knochenzementteig zu erhalten. Die Durmischung der beiden Ausgangskomponenten kann auf unterschiedliche Weise erfolgen. In einer Ausführungsform des Verfahrens erfolgt das Durchmischen unter aktiver Mitwirkung des Anwenders der Vorrichtung, beispielsweise unter Schütteln der Vorrichtung oder durch Betätigung eines Mischelements im vorderen Teil des Innenraums, insbesondere eines Rührers.

Eine Ausführungsform des Verfahrens ist dadurch gekennzeichnet, dass die Monomerflüssigkeit mit Hilfe eines hydrophilen Additivs in dem Knochenzementpulver verteilt wird. Ein Vorteil ist, dass dies ohne aktive Beteiligung des Anwenders der Vorrichtung vonstattengeht, was mögliche Fehler des Anwenders beim Mischen vermeidet. Ein möglicher Fehler ist, dass der Anwender nicht über die gesamte Länge des vorderen Teils des Innenraums mischt, so dass Teile des Knochenzementpulvers nicht mit Monomerflüssigkeit benetzt werden. Ein weiterer Vorteil ist, dass die Vorrichtung dadurch einfacher und mit weniger beweglichen Bauteilen ausgestaltet werden kann, was sowohl das Risiko für Fehlfunktionen als auch die Herstellungskosten der Vorrichtung verringert.

Die Vorrichtung ist dadurch gekennzeichnet, dass diese einen Knochenzementteig aus zwei Ausgangskomponenten bereitstellt. Unter einem Knochenzementteig wird eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Durch Aushärtung wird aus einem Knochenzementteig ein Knochenzement. Bevorzugt handelt es sich bei diesen Knochenzementen um Polymethylmethacrylat-Knochenzemente (PMMA Knochenzemente). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzementteig. Dabei wird eine Polymerisation des Monomers durch Radikale eingeleitet. Mit fortschreitender Polymerisation des Monomers erhöht sich die Viskosität des Knochenzementteigs, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulver zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver als hydrophiles Additiv mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin. Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

### Figuren

Die Erfindung wird im Folgenden durch Figuren weiter beispielhaft illustriert. Die Erfindung ist nicht auf die Figuren beschränkt.

Es zeigen
- Fig. 1: einen schematischen Längsschnitt einer beispielhaften Vorrichtung zum Bereitstellen eines Knochenzementteigs umfassend eine Mischeinheit und ein Reservoir mit einer mit einer Monomerflüssigkeit befüllten Ampulle,
- Fig. 2: die Vorrichtung aus Figuren 1, wobei ein erster Formschluss, ein zweiter Formschluss und ein Drehpunkt angedeutet sind,
- Fig. 3: eine perspektivische Seitenansicht der Mischeinheit der Vorrichtung aus den Figuren 1 und 2,
- Fig. 4: die Vorrichtung aus den Figuren 1 bis 3 bei einem fluidleitenden Öffnen der Ampulle und beim Fördern der Monomerflüssigkeit in die Mischeinheit,
- Fig. 5: die Vorrichtung aus den Figuren 1 bis 4, wobei das Reservoir von der Mischeinheit getrennt ist,
- Fig. 6: die Vorrichtung aus den Figuren 1 bis 5 mit einem fluidleitend geschlossenen Verschlusselement in Form einer Schraube,
- Fig. 7: die Vorrichtung aus den Figuren 1 bis 6 beim Fördern der Monomerflüssigkeit in das Knochenzementpulver,
- Fig. 8: die Vorrichtung aus den Figuren 1 bis 7 mit bereitgestelltem Knochenzementteig,
- Fig. 9: die Vorrichtung aus den Figuren 1 bis 8 beim Austragen des Knochenzementteigs,
- Fig. 10: einen schematischen Längsschnitt einer weiteren beispielhaften Vorrichtung zum Bereitstellen eines Knochenzementteigs umfassend eine Mischeinheit und ein Reservoir mit einer mit einer Monomerflüssigkeit befüllten Ampulle,
- Fig. 11: die Vorrichtung aus Figur 10 bei einem fluidleitenden Öffnen der Ampulle und beim Fördern der Monomerflüssigkeit in die Mischeinheit,
- Fig. 12: die Vorrichtung aus den Figuren 10 und 11 mit einem fluidleitend geschlossenen Verschlusselement in Form eines Drehventils, und
- Fig. 13: ein Flussdiagramm eines Verfahrens zum Bereitstellen eines Knochenzementteigs.

### Beschreibung der Figuren

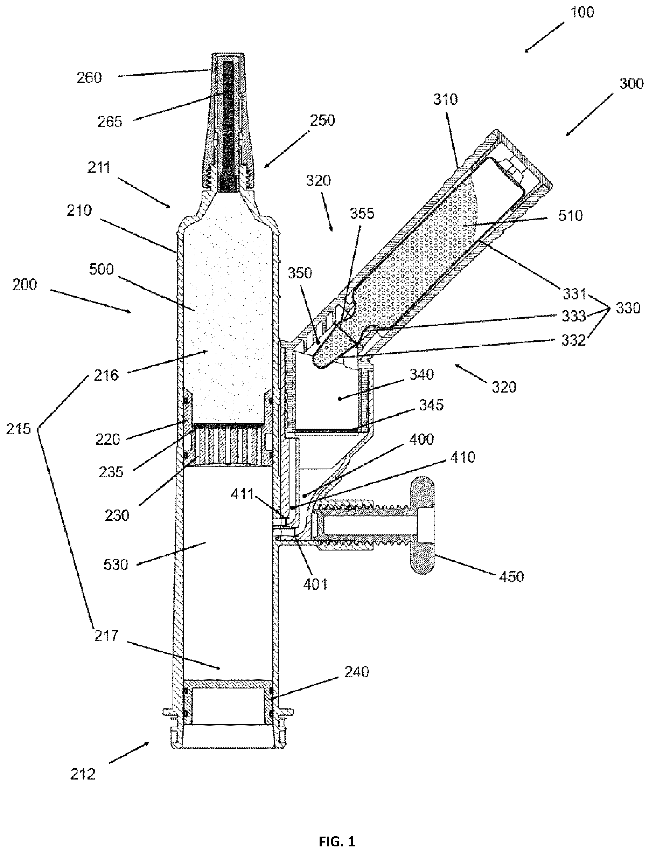

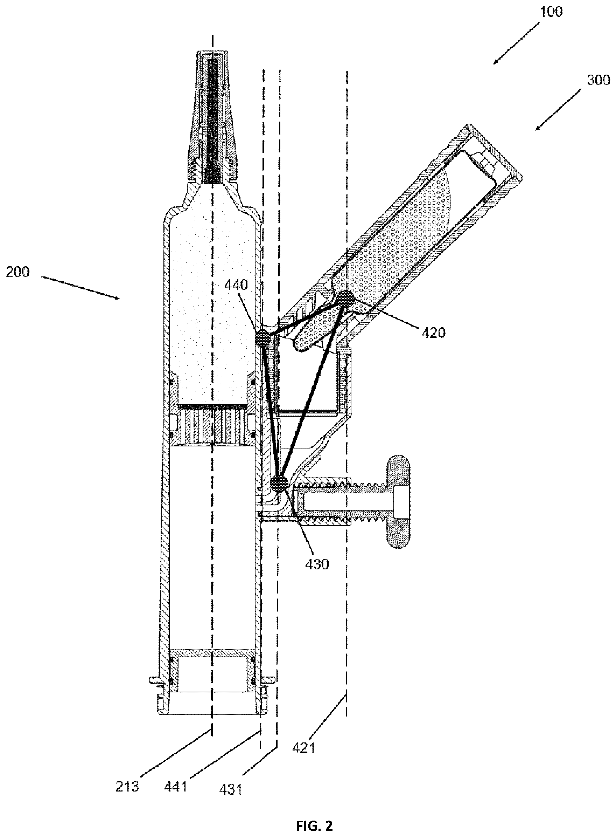

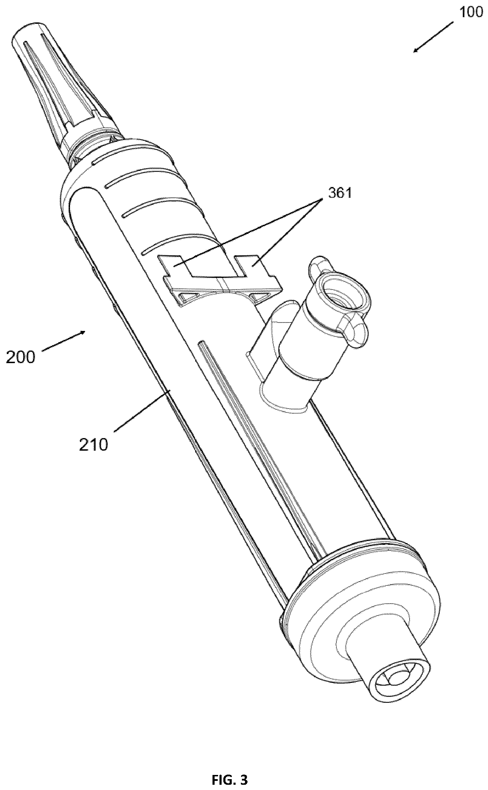

**Figur 1** zeigt einen schematischen Längsschnitt einer beispielhaften Ausführung einer Vorrichtung 100 zum Bereitstellen eines Knochenzementteigs aus zwei Ausgangskomponenten in einem Ausgangszustand. Die Vorrichtung 100 umfasst eine Mischeinheit 200 und ein Reservoir 300, welche reversibel trennbar über einen ersten Formschluss und einen zweiten Formschluss miteinander verbunden sind (vgl. Figuren 2 und 3).

Die Mischeinheit 200 ist rohrartig aufgebaut und umfasst eine hohlzylinderförmige Kartusche 210 mit einem Innenraum 215, welcher durch einen reversibel im Innenraum 215 axial verschiebbaren Austragskolben 220 in einen proximalen Teil 216 und einen distalen Teil 217 des Innenraums 215 unterteilt ist. Im proximalen Teil 216 des Innenraums 215 lagert ein Knochenzementpulver 500 als eine erste Ausgangskomponente des Knochenzementteigs und im Reservoir 300 lagert eine Ampulle 330, welche eine Monomerflüssigkeit 510 als eine zweite Ausgangskomponente des Knochenzementteigs beinhaltet. Das Knochenzementpulver 500 enthält als Hauptbestandteil partikuläres Polymethylmethacrylat sowie ein hydrophiles Additiv, mit dem die Monomerflüssigkeit 510 in dem Knochenzementpulver 500 mischeinrichtungsfrei verteilbar ist.

Der Austragskolben 220 ist für Feststoffe undurchlässig ausgestaltet, so dass kein Knochenzementpulver 500 vom proximalen Teil 216 in den distalen Teil 216 des Innenraums 215 gelangen kann. Der Austragskolben 220 weist ein Leitungsmittel 230 (lediglich exemplarisch gekennzeichnet) in Form mehrerer Durchlässe auf, durch welches zwischen dem proximalen Teil 216 und dem distalen Teil 217 des Innenraums 215 eine fluidleitende Verbindung ausgebildet ist. Das Leitungsmittel 230 ist durch ein Filtermittel 235 in Form einer Porenscheibe für Feststoffe oder Knochenzementteige undurchlässig verschlossen, wobei die Porenscheibe ein problemloses Fördern der Monomerflüssigkeit 510 aus dem distalen Teil 217 in den proximalen Teil 216 des Innenraums 215 erlaubt. In der gezeigten Ausführungsform der Vorrichtung 100 ist das Filtermittel 235 auf dem dem proximalen Teil 216 des Innenraums 215 zugewandten proximalen Ende des Leitungsmittels 230 angeordnet. In weiteren, nicht gezeigten, Ausführungsformen ist das Filtermittel 235 auf der dem distalen Teil 217 des Innenraums 215 zugewandten distalen Ende des Leitungsmittels 230 oder an beiden Enden des Leitungsmittels 230 angeordnet. Ein Vorteil eines wie gezeigt angeordneten Filtermittels 235 ist, dass der sich im proximalen Teil 216 des Innenraums 215 ausbildende Knochenzementteig das Leitungsmittel 230 nicht verstopfen kann.

Distal zum Austragskolben 220 ist innerhalb des distalen Teil 217 des Innenraums 215 ein innerhalb des Innenraums 215 axial beweglicher Förderkolben 240 angeordnet. Der Förderkolben 240 schließt ein distales Kartuschenende 212 der Kartusche 210 fluidleitend ab. Die Mischeinheit 200 weist ferner an einem, dem distalen Kartuschenende 212 gegenüberliegenden, proximalen Kartuschenende 211 eine Austragsöffnung 250 auf, die den dem Austragskolben 220 abgewandten Bereich des proximalen Teil 216 des Innenraums 215 der Kartusche 210 begrenzt. Die Austragsöffnung 250 ist im Ausgangszustand der Vorrichtung 100 durch eine Verschlusskappe 260 mit einen Stopfen 265 verschlossen, so dass kein Knochenzementpulver 500 aus der Kartusche 210 entweichen kann. Der Stopfen 265 ist gasdurchlässig ausgestaltet, um ein Gas 530, welches sich im Innenraum 215 vorhanden ist, durch das Knochenzementpulver 500 hindurch und aus der Vorrichtung 100 heraus transferieren zu können, bevor die Ausbildung des Knochenzementteigs einsetzt.

Das Reservoir 300 weist einen rohrartigen Reservoirbehälter 310 auf, in welchem zwei Ampullen 330, insbesondere zwei Glasampullen, nebeneinander lagern (lediglich eine der Ampullen 330 ist in der gezeigten Ansicht sichtbar). Die Ampullen 330 weisen jeweils einen Ampullenkörper 331, einen der Mischeinheit 200 zugewandten Ampullenkopf 332 und einen zwischen Ampullenkörper 331 und Ampullenkopf 332 liegende Ampullenhals 333, welcher als Sollbruchstelle für die Ampullen 330 fungiert, auf. In den Ampullen 330 lagert die Monomerflüssigkeit 510. Der Ampullenkopf 332 der Ampullen 330 ist abschnittsweise in einer Verbindung 350 angeordnet, welche einen Hohlraum 340 des Reservoirs 300 mit den Ampullen 330 verbindet. Die Verbindung 350 weist einen Verbindungsdurchmesser 355 auf, welcher etwa 5 % größer ist als ein Durchmesser der Ampullenköpfe 332, so dass die Verbindung 350 die Ampullenköpfe 332 gegen ein Verkippen innerhalb der Zeichenebene fixiert. Um ein Verkippen der Ampullen 330, insbesondere der Ampullenköpfe 332, gegen die Verbindung 350, wobei in der gezeigten Ausführungsform ein Verkippen in der Zeicheneben möglich ist, zu ermöglichen, weist der Reservoirbehälter 310 im Bereich eines Übergangs von Verbindung 350 zu Ampullenkörper 331 einen verformbaren Bereich 320 auf.

Innerhalb des Hohlraums 340 ist im Reservoir 300 ein Filterelement 345 angeordnet, so dass Bruchstücke der Ampullen 330 nach einem fluidleitenden Öffnen derselben nicht über den Hohlraum 340 in die Mischeinheit 200 gelangen können, sondern am Filterelement 345 zurückgehalten werden.

Das Reservoir 300, insbesondere der Hohlraum 340, ist über einen Einlasskanal 400 und einen Auslasskanal 410 fluidleitend mit der Mischeinheit 200, insbesondere dem distalen Teil 217 des Innenraums 215, verbunden. In der gezeigten Ansicht befindet sich der Einlasskanal 400 und der Auslasskanal 410 in einer ersten Kanalposition, so dass das Reservoir 300 und die Mischeinheit 200 fluidleitend über die beiden Kanäle 400, 410 miteinander verbunden sind. Der Einlasskanal 400 dient einem Einleiten der Monomerflüssigkeit 510 aus dem Reservoir 300 in die Mischeinheit. Der Auslasskanal dient einem Ausleiten des Gases 530 aus der Mischeinheit 200 in das Reservoir 300, welches durch die in den distalen Teil 217 des Innenraums 215 einfließende Monomerflüssigkeit 510 verdrängt wird. Um einen guten Stoffaustausch zwischen Reservoir 300 und Mischeinheit 200 zu gewährleisten, weist der Auslasskanal 410 in der gezeigten Ausführungsform einen minimalen

Auslasskanaldurchmesser 411 auf, welcher einem minimalen Einlasskanaldurchmesser 401 des Einlasskanals 400 entspricht. Der Einlasskanal 400 ist an einem den Ampullen 330 zugewandten Ende als Trichter ausgeformt, um die Monomerflüssigkeit 510 bei einem Ausfließen aus den zu öffnenden Ampullen 330 verbessert aufnehmen zu können.

Sowohl der Einlasskanal 400 als auch der Auslasskanal 410 sind zweiteilig ausgebildet, wobei ein der Mischeinheit 200 zugewandter Teil des Einlasskanals 400 und des Auslasskanals 410 jeweils als Durchführung in der Kartusche 210 ausgeformt ist und ein den Ampullen 330 zugewandter Teil des Einlasskanals 400 und des Auslasskanals 410 sich jeweils innerhalb des reversibel von der Mischeinheit 200 abtrennbaren Reservoirs 300 erstreckt. Der Einlasskanal 400 und der Auslasskanal 410 werden somit aus zwei Bauteilen der Vorrichtung 100 ausgeformt.

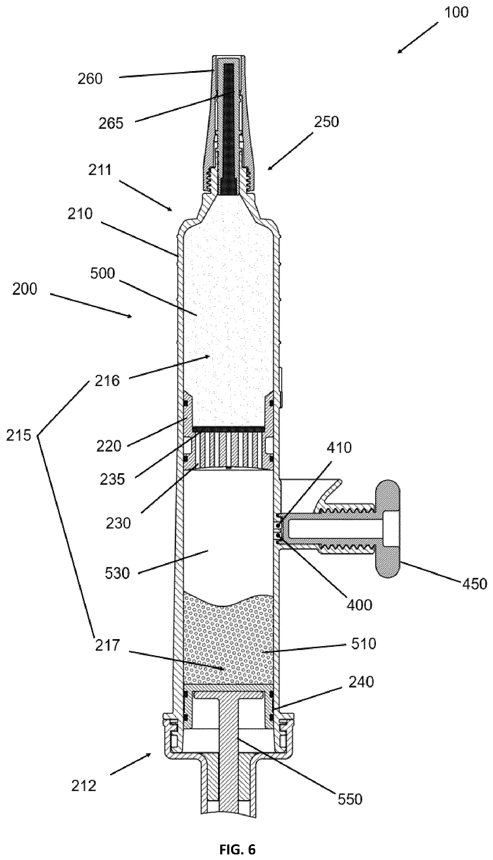

Die Vorrichtung 100 umfasst weiterhin ein Verschlusselement 450 in Form einer Schraube, welches nach einem Trennen von Mischeinheit 200 und Reservoir 300 die der Mischeinheit 200 zugewandten Teil des Einlasskanals 400 und des Auslasskanals 410, also die Durchführungen in der Kartusche 210, fluidleitend verschließen kann (vgl. Figur 6).

**Figur 2** zeigt die Vorrichtung 100 aus Figur 1, wobei der erste Formschluss 430, der zweite Formschluss 440 und ein Drehpunkt 420, um welchen die Ampullen 330 aufgrund des verformbaren Bereichs 320 durch Verkippen gegen die Verbindung 350 (vgl. Figur 1) drückbar sind, durch gefüllte Kreise angedeutet sind.

Der erste Formschluss 430 ist durch die Bauteile, welchen den Einlasskanal 400 und den Auslasskanal 410 (vgl. Figur 1) ausbilden, im Bereich des Einlasskanals 400 und des Auslasskanals 410 ausgeformt. Der zweite Formschluss 430 ist durch eine Spange (nicht gezeigt in Figur 1 und 2; vgl. Figur 3) ausgebildet.

Der erste Formschluss 430, der zweite Formschluss 440 und der Drehpunkt 420 bilden in der gezeigten Seitenansicht der Vorrichtung 100 ein Dreieck (angedeutet durch Verbindungslinien zwischen den gefüllten Kreisen) aus. Erfolgt ein Verkippen der Ampullen 330 um den Drehpunkt 420, so dass die Ampullen 330, insbesondere die Ampullenköpfe 332 (vgl. Figur 1), gegen die Verbindung 350 (vgl. Figur 1) gedrückt werden, so verteilt sich die dabei zum fluidleitenden Öffnen der Ampullen 330 benötige Kraft auf den ersten Formschluss 430 und den zweiten Formschluss 440. Durch die Anordnung des ersten Formschlusses 430, des zweiten Formschlusses 440 und des Drehpunkts 420 in Form eines Dreiecks wird dabei eine vorteilhafte Kraftverteilung erreicht. Insbesondere kann dadurch die Gefahr eines Abknickens oder Abbrechens des Einlasskanals 400 und des Auslasskanales 410 verringert werden.

Der erste Formschluss 430, der zweite Formschluss 440 und der Drehpunkt 420 sind so zueinander angeordnet, dass der zweite Formschluss 440 einen kürzeren Abstand zum ersten Formschluss 430 als der Drehpunkt 420 aufweist. Der Drehpunkt 420 und der erste Formschluss 430 sind somit weiter voneinander beabstandet als der erste Formschluss 430 und der zweite Formschluss 440. Dies sorgt für eine verbesserte Kraftverteilung der beim Verkippen zum Öffnen der Ampullen 330 um den Drehpunkt 420 benötigten Kraft auf die zwei Formschlüsse 430, 440 als auch gleichzeitig für eine möglichst platzsparende Ausgestaltung der Vorrichtung 100. Letzteres erleichtert insbesondere die Handhabung der Vorrichtung 100 durch einen Anwender.

Der erste Formschluss 430 liegt auf einer parallel zu einer Längsachse 213 der Kartusche 210 verlaufenden Geraden 431, der zweite Formschluss 440 liegt auf einer weiteren parallel zur Längsachse 213 der Kartusche 210 verlaufenden Geraden 441 und der Drehpunkt 420 liegt auf einer weiteren parallel zur Längsachse 213 der Kartusche 210 liegenden Geraden 421, wobei die Geraden 431, 441, 421 alle einen unterschiedlichen Geradenabstand zur Längsachse 213 der Kartusche 210 aufweisen. In der gezeigten Ausführungsform ist der Geradenabstand zwischen der Geraden 421 durch den Drehpunkt 420 und der Längsachse 213 am größten, gefolgt vom Geradenabstand zwischen der Geraden 431 durch den ersten Formschluss 430 und der Längsachse 213. Die unterschiedlichen Geradenabstände verbessern die Kraftverteilung der beim Verkippen zum Öffnen der Ampullen 330 um den Drehpunkt 420 benötigten Kraft auf die zwei Formschlüsse 430, 440 und erlauben gleichzeitig eine möglichst platzsparende Ausgestaltung der Vorrichtung 100. Letzteres erleichtert insbesondere die Handhabung der Vorrichtung 100 durch einen Anwender.

**Figur 3** zeigt eine perspektivische Seitenansicht der Mischeinheit 200 der Vorrichtung 100 aus den Figuren 1 und 2. Die Mischeinheit 200 weist an einer Außenseite der Kartusche 210 zwei Spangenausstülpungen 261 auf, welche reversibel in Spangeneinkerbungen an einer Außenfläche des Reservoirs 300 (nicht gezeigt) einschiebbar sind, um so eine Spange (nicht gezeigt) formen, welche den zweiten Formschluss (vgl. Figur 2) ausbildet.

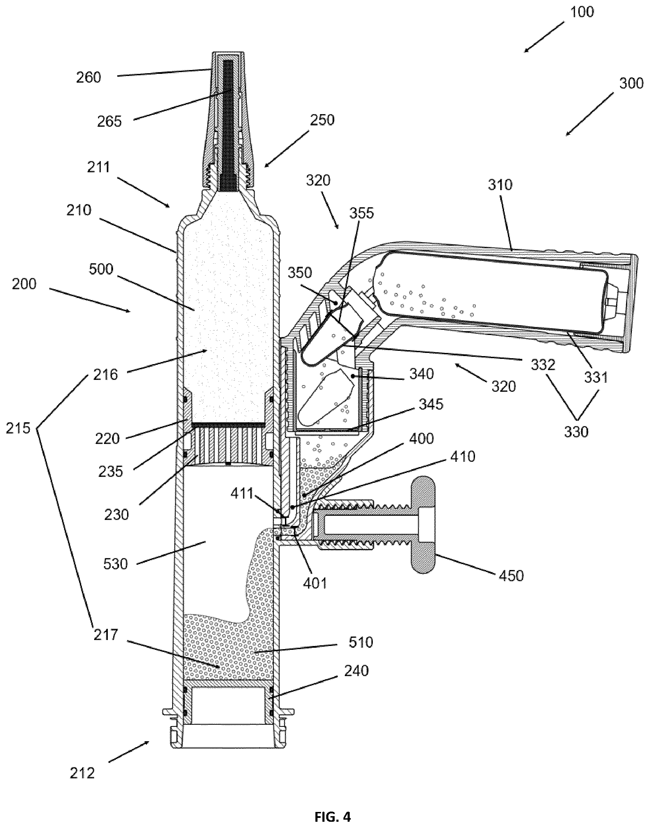

**Figur 4** zeigt die Vorrichtung 100 aus den Figuren 1 bis 3 mit um den Drehpunkt 420 (vgl. Figur 2) verkippten Ampullen 330. Dazu ist der Reservoirbehälter 310 am verformbaren Bereich 320 abgeknickt, so dass die Ampullenköpfe 332 gegen die Verbindung 350 gepresst und die Ampullen 330 im Bereich des Ampullenhalses 332 (vgl. Figur 1) fluidleitend geöffnet wurden. Die in den fluidleitend geöffneten Ampullen 330 gelagerte Monomerflüssigkeit 510 ist bereits größtenteils aus den Ampullen 330 über den Hohlraum 340 und den Einlasskanal 400 in den distalen Teil 217 des Innenraums 215 ausgeflossen. Der Ampullenkopf 332 einer der Ampullen 330 ist vollständig aus der Verbindung 350 in den Hohlraum 340 übergegangen. Dabei wurde der Ampullenkopf 332 vom Filterelement 345 aufgefangen, so dass dieser, oder Bruchstücke davon, nicht zum oder durch den Einlasskanal 400 gelangen kann, beziehungsweise können. Der Hohlraum 340 ist so dimensioniert, dass der Ampullenkopf 332 vollständig drehbar in demselben lagerbar ist, so dass eventuell noch im Ampullenkopf 332 nach dem Öffnen der Ampulle 330 vorhandene Monomerflüssigkeit 510 in den Hohlraum 340 ausfließen kann. Dies ist in Figur 4 bereits geschehen. Gleichzeitig ist ein Teil des Gases 530 aus dem Innenraum 215 über den Auslasskanal 410 in das Reservoir 300 ausgeleitet worden. Das Volumen des ausgeleiteten Teils des Gases 530 entspricht dabei im Wesentlichen dem Volumen der bereits in den distalen Teil 217 des Innenraums 215 eingeleiteten Monomerflüssigkeit 510.

Um die Monomerflüssigkeit 510 im Wesentlichen vollständig durch den Einlasskanal 400, und nicht über den Auslasskanals 410, in die Mischeinheit 200 zu befördern, weist der Einlasskanal 400 einen geringeren Abstand zum Drehpunkt 420 auf als der Auslasskanal 410. Weiterhin mündet der Auslasskanal 410 proximal zum Einlasskanal 400 in den Innenraum 215, so dass die einfließende Monomerflüssigkeit 510 ein Ausleiten des Gases 530 aus dem Innenraum 215 durch den Auslasskanal 410 nicht behindert.

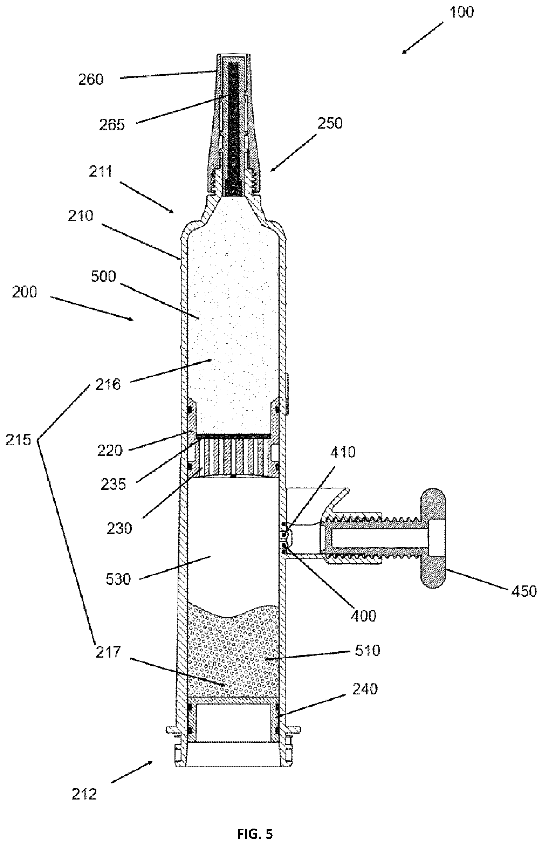

**Figur 5** zeigt die Vorrichtung 100 aus den Figuren 1 bis 4, wobei das Reservoir 300 durch Lösen des ersten Formschlusses 430 und des zweiten Formschlusses 440 nach einem im Wesentlichen vollständigen Einfließen der Monomerflüssigkeit 510 in den distalen Teil 217 des Innenraums 215 von der Mischeinheit 200 abgetrennt wurde. Durch Abtrennen des Reservoirs 300 wurden die dem Reservoir 300 zugewandten Teil des Einlasskanals 400 und des Auslasskanals 410 von den der Mischeinheit 200 zugewandten Teilen des Einlasskanals 400 und des Auslasskanals 410 fluidleitend getrennt. Der Einlasskanal 400 und der Auslasskanal 410 befinden sich somit in einer fluidleitend getrennten zweiten Kanalposition.

Über den der Mischeinheit 200 zugewandten Teil des Einlasskanals 400 und des Auslasskanals 410, also den Durchführungen in der Kartusche 210, ist der distale Teil 217 des Innenraums 215 fluidleitend mit der Umgebung der Vorrichtung 100 verbunden. Ein Vortreiben des Förderkolbens 240 in Richtung des Austragskolbens 220 würde in der gezeigten Ausführungsform der Vorrichtung 100 somit die Monomerflüssigkeit 510 zumindest teilweise aus der Mischeinheit 200 austragen, anstelle diese, wie gewünscht, im Wesentlichen vollständig durch das Leitungsmittel 230 in den proximalen Teil 216 des Innenraums 215 fördern, um dort zusammen mit dem Knochenzementpulver 500 den Knochenzementteig auszubilden.

**Figur 6** zeigt die Vorrichtung 100 aus den Figuren 1 bis 6, wobei das Verschlusselement 450 bis zum Kontakt mit der Kartusche 210 eingeschraubt ist und somit in eine Verschlussposition verbracht wurde, in der der der Mischeinheit 200 zugewandte Teil des Einlasskanals 400 und des Auslasskanals 410 fluidleitend gegen die Umgebung der Vorrichtung 100 verschlossen ist. In der Verschlussposition ermöglicht das Verschlusselement 450 ein im Wesentlichen vollständig Fördern der Monomerflüssigkeit 510 in den proximalen Teil 216 des Innenraums durch ein Vortreiben des Förderkolbens 240 in Richtung des Austragskolbens 220.

Um das Fördern der Monomerflüssigkeit 510 in den proximalen Teil 216 des Innenraums 215 für den Anwender zu erleichtern, ist das distale Kartuschenende 212 mit einer Austragshilfe 550 in Form einer Austragspistole verbunden, welche distal gegen den Förderkolben 240 drücken kann, um diesen proximal zu verschieben.

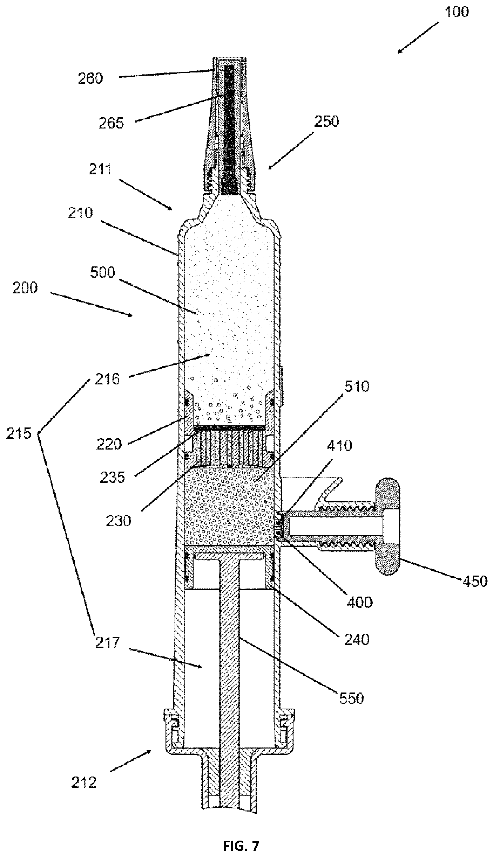

**Figur 7** zeigt die Vorrichtung 100 aus den Figuren 1 bis 6 beim Fördern der Monomerflüssigkeit 510 aus dem distalen Teil 217 des Innenraums 215 durch das Leitungsmittel 230 in den proximalen Teil 216 des Innenraums 215. Aufgrund des Verschlussmittels 450 in der Verschlussposition wird dabei keine Monomerflüssigkeit 510 aus dem distalen Teil 217 des Innenraums 215 an die Umgebung der Vorrichtung 100 abgegeben.

Mit beginnendem Kontakt der Monomerflüssigkeit 510 mit dem Knochenzementpulver 500 kommt es im proximalen Teil 216 des Innenraums 215 zur Ausbildung des Knochenzementteigs. Das hydrophile Additiv im Knochenzementpulver 500 sorgt dabei für eine verbesserte Verteilung der Monomerflüssigkeit 510 im Knochenzementpulver 500, so dass eine Bereitstellung eines homogenen Knochenzementteigs ohne mechanische Hilfsmittel, wie beispielsweise einen Mischstab, und somit mischeinrichtungsfrei möglich ist.

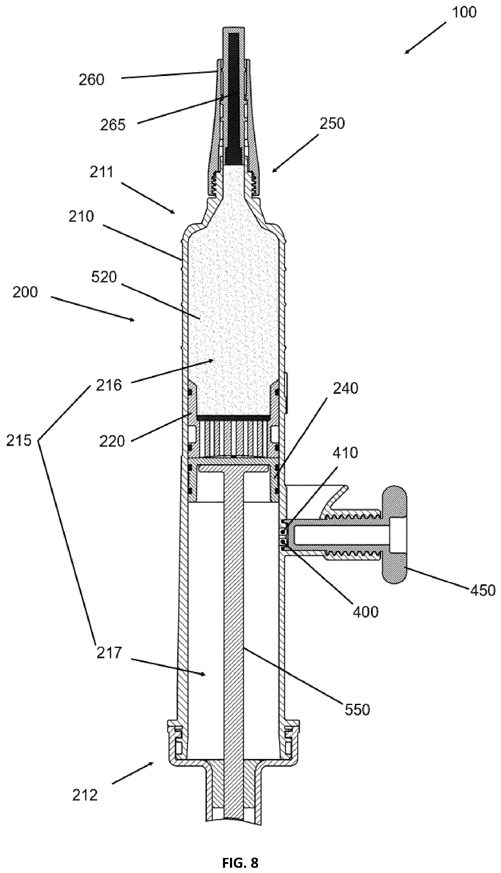

**Figur 8** zeigt die Vorrichtung 100 aus den Figuren 1 bis 7 mit im proximalen Teil 216 des Innenraums 215 bereitgestelltem Knochenzementteig 520. Der Förderkolben 240 wurde dazu proximal durch die Austragshilfe 550 verschoben, bis dieser distal am Austragskolben 220 anliegt. Dies hat die Monomerflüssigkeit 510 im Wesentlichen vollständig in den proximalen Teil 216 des Innenraums 215 gefördert.

Durch die Ausbildung des Knochenzementteigs 520 ist es zu einem Aufquellen des Knochenzementpulvers 500 gekommen, was mit einer Vergrößerung des Volumens einhergeht. Dadurch wurde der Stopfen 265 abschnittsweise aus der Verschlusskappe 260 ausgebracht, was dem Anwender der Vorrichtung 100 die Bereitstellung des Knochenzementteigs 520 signalisiert.

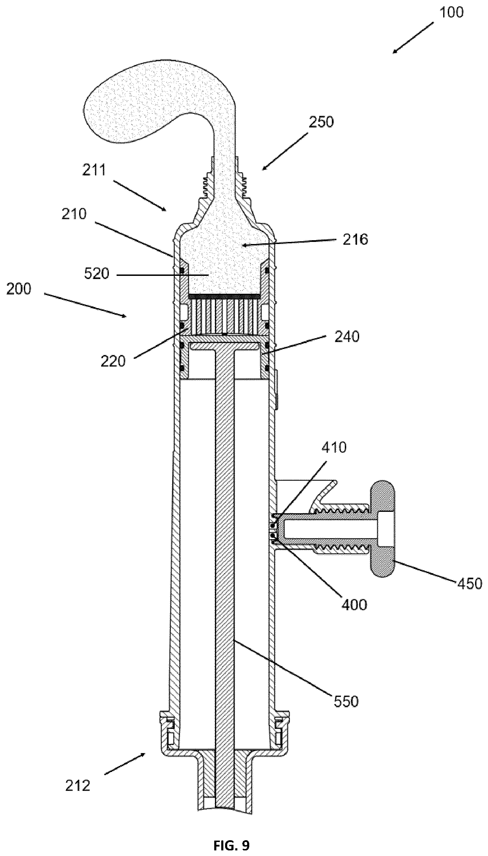

**Figur 9** zeigt die Vorrichtung 100 aus den Figuren 1 bis 8 beim Austragen des Knochenzementteigs 520 aus der Austragsöffnung 250. Dazu wurde der Förderkolben 240 mitsamt Austragskolben 220 durch ein fortgesetztes Vortreiben der Austragshilfe 550 in Richtung der Austragsöffnung 550 vorgetrieben. In weiteren, nicht gezeigten Ausführungsform der Vorrichtung 100 kann die Austragsöffnung 250 mit einem Austragsschnorchel versehen sein, um ein zielgerichtetes Austragen des Knochenzements 520 zu unterstützen.

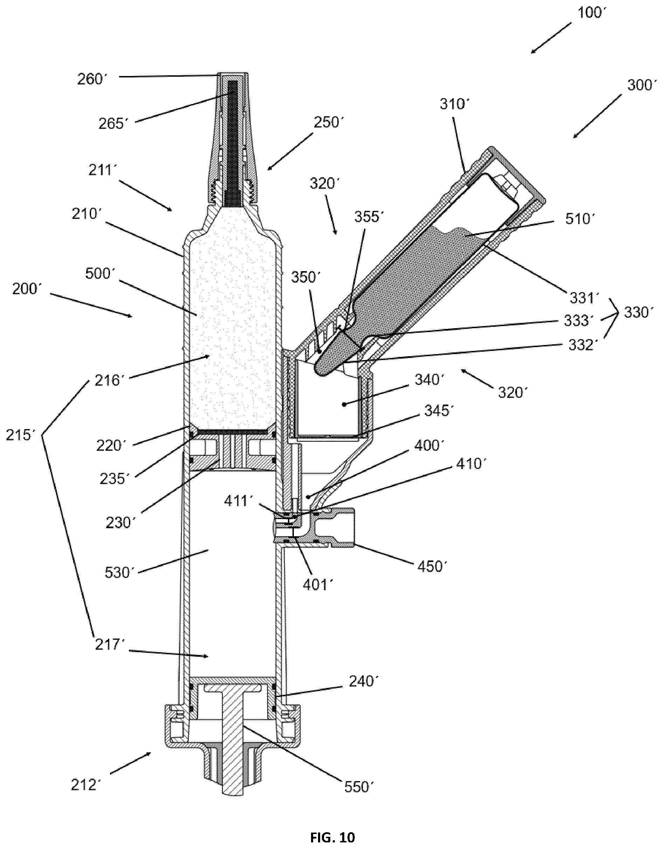

**Figur 10** zeigt eine weitere beispielhafte Ausführungsform einer Vorrichtung 100' zum Bereitstellen eines Knochenzementteigs aus zwei Ausgangskomponenten in einem Ausgangszustand. Die Ausführungsform der Vorrichtung 100' stimmt weitgehend mit der vorstehend beschriebenen und in den Figuren 1 bis 9 dargestellten Ausführungsform überein, so dass zur Vermeidung von Wiederholungen auf die vorstehende Beschreibung verwiesen wird. Abwandlungen einer gegenüber der in den Figuren 1 bis 9 gezeigten Ausführungsform weisen dasselbe Bezugszeichen mit einem Apostroph auf.

Die Vorrichtung 100' unterscheidet sich von der Vorrichtung 100 aus den Figuren 1 bis 9 durch ein Verschlusselement 450' in Form eines Drehventils. Der Einlasskanal 400' und der Auslasskanal 410' sind, ebenso wie bei der Vorrichtung 100 aus den Figuren 1 bis 9, zweiteilig ausgestaltet, wobei der der Mischeinheit 200' zugewandte Teil der beiden Kanäle 400', 410' sich jeweils durch das Verschlusselement 450' erstreckt. In Figur 10 befindet sich das Verschlusselement 450' in einer ersten Drehventilposition, so dass der Einlasskanal 400' und der Auslasskanal 410' sich in der ersten Kanalposition befinden, welche das Reservoir 300' und die Mischeinheit 200' fluidleitend miteinander verbindet.

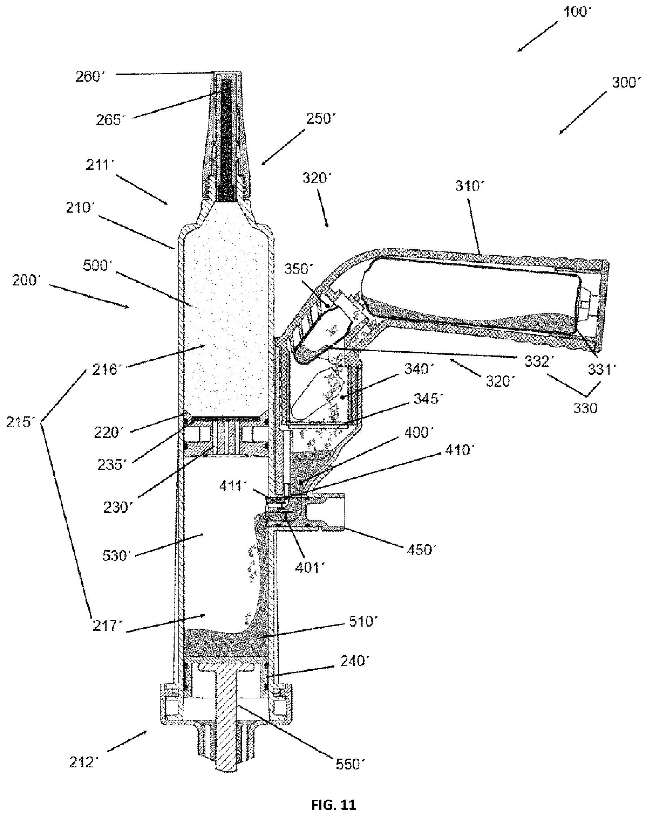

**Figur 11** zeigt die Vorrichtung 100' aus Figur 10 mit fluidleitend geöffneten Ampullen 330'. Dazu ist der Reservoirbehälter 310' am verformbaren Bereich 320' abgeknickt, so dass die Ampullenköpfe 332' gegen die Verbindung 350' gepresst und die Ampullen 330' im Bereich des Ampullenhalses 332' (vgl. Figur 10) fluidleitend geöffnet wurden. Die in den fluidleitend geöffneten Ampullen 330' gelagerte Monomerflüssigkeit 510' ist bereits größtenteils aus den Ampullen 330' über den Hohlraum 340' und den Einlasskanal 400' in den distalen Teil 217' des Innenraums 215' ausgeflossen. Der Ampullenkopf 332' einer der Ampullen 330' ist vollständig aus der Verbindung 350' in den Hohlraum 340' übergegangen. Dabei wurde der Ampullenkopf 332' vom Filterelement 345' aufgefangen, so dass dieser, oder Bruchstücke davon, nicht zum oder durch den Einlasskanal 400' gelangen kann, beziehungsweise können. Der Hohlraum 340' ist so dimensioniert, dass der Ampullenkopf 332' vollständig drehbar in demselben lagerbar ist, so dass eventuell noch im Ampullenkopf 332' nach dem Öffnen der Ampulle 330' vorhandene Monomerflüssigkeit 510' in den Hohlraum 340' ausfließen kann. Dies ist in Figur 11 bereits geschehen. Gleichzeitig ist ein Teil des Gases 530' aus dem Innenraum 215' über den Auslasskanal 410' in das Reservoir 300' ausgeleitet worden. Das Volumen des ausgeleiteten Teils des Gases 530' entspricht dabei im Wesentlichen dem Volumen der bereits in den distalen Teil 217' des Innenraums 215' eingeleiteten Monomerflüssigkeit 510'.

Um die Monomerflüssigkeit 510' im Wesentlichen vollständig durch den Einlasskanal 400', und nicht über den Auslasskanals 410', in die Mischeinheit 200' zu befördern, weist der Einlasskanal 400' einen geringeren Abstand zum Drehpunkt 420' auf als der Auslasskanal 410'. Weiterhin mündet der Auslasskanal 410' in der ersten Drehventilposition proximal zum Einlasskanal 400' in den Innenraum 215', so dass die einfließende Monomerflüssigkeit 510' ein Ausleiten des Gases 530' aus dem Innenraum 215' durch den Auslasskanal 410' nicht behindert.

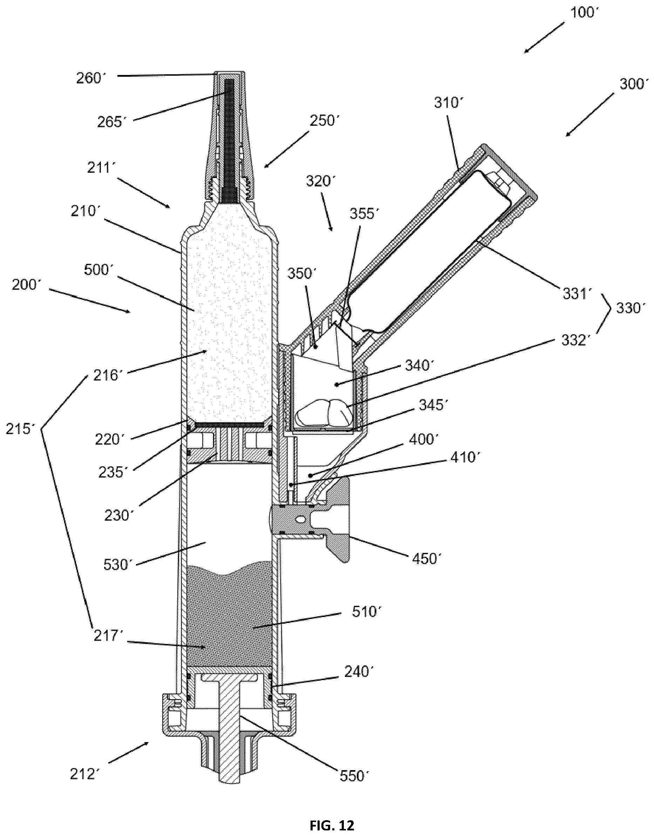

**Figur 12** zeigt die Vorrichtung 100' aus den Figuren 10 und 11 mit im Wesentlichen vollständig in den distalen Teil 217' des Innenraums 215' geförderter Monomerflüssigkeit 510'. Um ein im Wesentlichen vollständiges Fördern der Monomerflüssigkeit 510' aus dem distalen Teil 217' des Innenraums 215' der gezeigten Ausführungsform der Vorrichtung 100' zu erleichtern, ist das Verschlusselement 450' in Form eines Drehventils in eine zweite Drehventilposition durch eine Drehung um 90° um eine Achse des Verschlusselements 450' verbracht worden. Der Einlasskanal 400' und der Auslasskanal 410' befinden sich in der zweiten Drehventilposition in einer zweiten Kanalposition, in welcher die Mischeinheit 200' und das Reservoir 300' fluidleitend getrennt voneinander sind. Das Fördern der Monomerflüssigkeit 510' in den proximalen Teil 216' des Innenraums 215' sowie das Bereitstellen und Austragen des Knochenzementteigs kann analog zur Vorrichtung 100 wie in den Figuren 7 bis 9 gezeigt durchgeführt werden.

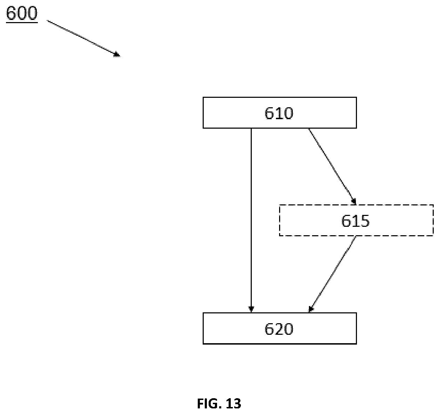

**Figur 13** zeigt ein Verfahren 600 zur Bereitstellung eines Knochenzementteigs 520 aus zwei Ausgangskomponenten mittels den Vorrichtungen 100, 100' gemäß den Figuren 1 bis 9 und 10 bis 12 umfassend die Schritte 610 und 620 sowie optional Schritt 615.

In einem Schritt 610 fließt die Monomerflüssigkeit 510, 510' aus dem Reservoir 300, 300' durch den Einlasskanal 400, 400' in den distalen Teil 217, 217' des Innenraums 215, 215' der Mischeinheit 200, 200'. Gleichzeitig verdrängt die in den distalen Teil 217, 217' des Innenraums 215, 215' einfließende Monomerflüssigkeit 510, 510' entsprechend ihrem Volumen das im Innenraum 215, 215' vorhandene Gas 530, 530', welches gleichzeitig mit dem Einfließen der Monomerflüssigkeit 510, 510' aus dem Innenraum 215, 215' in das Reservoir ausgeleitet wird. Der Einlasskanal 400, 400' wirkt somit mit dem Auslasskanal 410, 410' synergistisch zusammen, um einen Stoffaustausch zwischen Reservoir 300, 300' und Mischeinheit 200, 200' zu verbessern.

In einem Schritt 620 wird die Monomerflüssigkeit 510, 510' aus dem distalen Teil 217, 217' des Innenraums 215, 215' durch das Leitungsmittel 230, 230' in den proximalen Teil 216, 216' des Innenraums 215, 215' gefördert. Dazu wird der Förderkolben 240, 240' in Richtung des Austragskolben 220, 220' innerhalb des Innenraums 215, 215' vorgetrieben. Um die Monomerflüssigkeit 510, 510' im Wesentlichen vollständig in den proximalen Teil 216, 216' des Innenraums 215, 215' zu fördern, wird der Förderkolben 240, 240' vorzugsweise in Richtung des Austragskolbens 220, 220' vorgetrieben, bis dieser distal am Austragskolben 220, 220' anliegt.

In einer bevorzugten Ausführungsform des Verfahrens 600 wird in einem Schritt 615, welcher zwischen Schritt 610 und Schritt 620 stattfindet, der der Mischeinheit 200, 200' zugwandte Teil des zweiteiligen Einlasskanals 400, 400' und des zweiteiligen Auslasskanal 410, 410' durch das Verschlusselement 450, 450' fluidleitend verschlossen. Dies erleichtert ein im Wesentlichen vollständiges Fördern 620 der Monomerflüssigkeit 510, 510' in den proximalen Teil 216, 216' des Innenraums 215, 215', da die Monomerflüssigkeit 510, 510' nicht versehentlichen durch Vortreiben des Förderkolbens 240, 240' aus den entsprechenden Teilen der Kanäle 400, 400'. 410, 410' aus der Vorrichtung 100, 100' ausgetragen werden kann.

Vorzugsweise wird vor Schritt 620 das Reservoir 300, 300' durch Lösen des ersten Formschlusses 430 und des zweiten Formschlusses 440 von der Mischeinheit 200, 200' abgetrennt, was die Handhabung der Vorrichtung für einen Anwender verbessert.

Mit dem Fördern 620 der Monomerflüssigkeit 510, 510' in den proximalen Teil 216, 216' des Innenraums 215, 215' kommt es zur Ausbildung des Knochenzementteigs 520 aus dem Knochenzementpulver 500, 500' und der Monomerflüssigkeit 510, 510'. Vorzugsweise findet die Ausbildung des Knochenzementteigs 520 ohne mechanische Einwirkung durch den Anwender der Vorrichtung 100, 100' statt. Das Verfahren 600 wird somit vorzugsweise ohne mechanische Einwirkung, beispielsweise ohne Betätigung einer Mischeinrichtung, wie beispielsweise eine Mischstabs, durchgeführt.

Nach dem Bereitstellen des Knochenzementteigs 520 wird dieser vorzugsweise durch ein fortgesetztes Vortreiben des Förderkolbens 240, 240' in Richtung der Austragsöffnung 250, 250' aus der Vorrichtung 100, 100', insbesondere aus dem proximalen Teil 216, 216' des Innenraums 215, 215', ausgetragen. Dabei wirkt der Förderkolben 240, 240' aus distaler Richtung auf den Austragskolben 220, 220' ein, welcher dabei in Richtung der Austragsöffnung 250, 250' verbracht wird. Das Fördern 620 der Monomerflüssigkeit 510, 510' und das Austragen des Knochenzementteigs 520 erfolgt somit vorzugsweise mittels einem unidirektionalen Vortreiben des Förderkolbens 240, 240'.

### Bezugszeichen

- 100, 100': Vorrichtung
- 200. 200': Mischeinheit
- 210, 210': hohlzylinderförmige Kartusche
- 211, 211': proximales Kartuschenende
- 212, 212': distales Kartuschenende
- 213: Längsachse der Kartusche
- 215, 215': Innenraum der Kartusche
- 216, 216': proximaler Teil des Innenraums
- 217, 217': distaler Teil des Innenraums
- 220, 220': Austragskolben
- 230, 230': Leitungsmittel
- 235, 235': Filtermittel
- 240, 240': Förderkolben
- 250, 250': Austragsöffnung
- 260, 260': Verschlusskappe
- 265, 265': Stopfen
- 300, 300': Reservoir
- 310, 310': Reservoirbehälter
- 320, 320': verformbarer Bereich
- 330, 330': Ampulle
- 331, 331': Ampullenkörper
- 332, 332': Ampullenkopf
- 333, 333': Ampullenhals
- 340, 340': Hohlraum
- 345, 345': Filterelement
- 350, 350': Verbindung
- 355, 355': Verbindungsdurchmesser
- 361: Spangenausstülpungen
- 400, 400': Einlasskanal
- 401, 401': Einlasskanaldurchmesser
- 410, 410': Auslasskanal
- 411, 411': Auslasskanaldurchmesser
- 420: Drehpunkt
- 421: Gerade durch den Drehpunkt
- 430: erster Formschluss
- 431: Gerade durch den ersten Formschluss
- 440: zweiter Formschluss
- 441: Gerade durch den zweiten Formschluss
- 450, 450': Verschlusselement
- 500, 500': Knochenzementpulver
- 510, 510': Monomerflüssigkeit
- 520: Knochenzementteig
- 530, 530': Gas
- 550, 550': Austragshilfe
- 600: Verfahren
- 610: Fließen
- 615: Verschließen
- 620: Fördern

## Patentansprüche

1. Vorrichtung (100, 100') zum Bereitstellen eines Knochenzementteigs (520) aus zwei Ausgangskomponenten, umfassend
eine Mischeinheit (200, 200') aufweisend eine hohlzylinderförmige Kartusche (210, 210') mit einem Innenraum (215, 215'), wobei in dem Innenraum (215, 215') ein axial im Innenraum (215, 215') beweglicher Austragskolben (220, 220') angeordnet ist, welcher den Innenraum (215, 215') in einen proximalen Teil (216, 216') des Innenraums (215, 215') und einen distalen Teil (217, 217') des Innenraums (215, 215') unterteilt, wobei der proximale Teil (216, 216') und der distale Teil (217, 217') des Innenraums (215, 215') über ein Leitungsmittel (230, 230') fluidleitend miteinander verbunden sind,
wobei im proximalen Teil (216, 216') des Innenraums (215, 215') ein Knochenzementpulver (500, 500') als erste Ausgangskomponente lagert und wobei im distalen Teil (217, 217') des Innenraums (215, 215') ein axial im Innenraum (215, 215') beweglicher Förderkolben (240, 240') angeordnet ist,
und ein Reservoir (300, 300') für eine Monomerflüssigkeit (510, 510') als zweite Ausgangskomponente, welches über einen Einlasskanal (400, 400') mit dem distalen Teil (217, 217') des Innenraums (215, 215') zum Einleiten der Monomerflüssigkeit (510, 510') aus dem Reservoir (300, 300') in die Mischeinheit (200, 200') fluidleitend verbunden oder verbindbar ist,
wobei
das Reservoir (300, 300') und die Mischeinheit (200, 200') über einen Auslasskanal (410, 410') fluidleitend verbunden oder verbindbar sind, über welchen ein Gas (530, 530') aus dem Innenraum (215, 215') in das Reservoir (300, 300') ausleitbar ist,
wobei der Einlasskanal (400, 400') und der Auslasskanal (410, 410') zumindest zweiteilig ausgeformt sind, so dass die Mischeinheit (200, 200') und das Reservoir (300, 300') in einer ersten Kanalposition des Einlasskanals (400, 400') und des Auslasskanals (410, 410') fluidleitend miteinander verbunden sind und in einer zweiten Kanalposition des Einlasskanals (400, 400') und des Auslasskanals (410, 410') fluidleitend getrennt voneinander sind,
wobei die Vorrichtung (100, 100') ein Verschlusselement (450, 450') aufweist, welches zumindest einen der Mischeinheit (200, 200') zugewandten Teil des zweiteiligen Einlasskanals (400, 400') und des zweiteiligen Auslasskanals (410, 410') fluidleitend verschließt oder verschließbar macht,
**dadurch gekennzeichnet, dass** das Verschlusselement (450') ein Drehventil ist, durch welches sich der der Mischeinheit (200') zugewandte Teil des zweiteiligen Einlasskanals (400') und des zweiteiligen Auslasskanals (410') erstreckt und welches in einer ersten Drehventilposition den Einlasskanal (400) und den Auslasskanal (410') in der ersten Kanalposition belässt und durch ein Drehen in eine zweite Drehventilposition den Einlasskanal (400') und den Auslasskanal (410') in die zweite Kanalposition verbringt.

2. Vorrichtung (100, 100') zum Bereitstellen eines Knochenzementteigs (520) aus zwei Ausgangskomponenten, umfassend
eine Mischeinheit (200, 200') aufweisend eine hohlzylinderförmige Kartusche (210, 210') mit einem Innenraum (215, 215'), wobei in dem Innenraum (215, 215') ein axial im Innenraum (215, 215') beweglicher Austragskolben (220, 220') angeordnet ist, welcher den Innenraum (215, 215') in einen proximalen Teil (216, 216') des Innenraums (215, 215') und einen distalen Teil (217, 217') des Innenraums (215, 215') unterteilt, wobei der proximale Teil (216, 216') und der distale Teil (217, 217') des Innenraums (215, 215') über ein Leitungsmittel (230, 230') fluidleitend miteinander verbunden sind,
wobei im proximalen Teil (216, 216') des Innenraums (215, 215') ein Knochenzementpulver (500, 500') als erste Ausgangskomponente lagert und wobei im distalen Teil (217, 217') des Innenraums (215, 215') ein axial im Innenraum (215, 215') beweglicher Förderkolben (240, 240') angeordnet ist,
und ein Reservoir (300, 300') für eine Monomerflüssigkeit (510, 510') als zweite Ausgangskomponente, welches über einen Einlasskanal (400, 400') mit dem distalen Teil (217, 217') des Innenraums (215, 215') zum Einleiten der Monomerflüssigkeit (510, 510') aus dem Reservoir (300, 300') in die Mischeinheit (200, 200') fluidleitend verbunden oder verbindbar ist,
wobei das Reservoir (300, 300') und die Mischeinheit (200, 200') über einen Auslasskanal (410, 410') fluidleitend verbunden oder verbindbar sind, über welchen ein Gas (530, 530') aus dem Innenraum (215, 215') in das Reservoir (300, 300') ausleitbar ist,
wobei der Einlasskanal (400, 400') und der Auslasskanal (410, 410') zumindest zweiteilig ausgeformt sind, so dass die Mischeinheit (200, 200') und das Reservoir (300, 300') in einer ersten Kanalposition des Einlasskanals (400, 400') und des Auslasskanals (410, 410') fluidleitend miteinander verbunden sind und in einer zweiten Kanalposition des Einlasskanals (400, 400') und des Auslasskanals (410, 410') fluidleitend getrennt voneinander sind,
wobei die Vorrichtung (100, 100') ein Verschlusselement (450, 450') aufweist, welches zumindest einen der Mischeinheit (200, 200') zugewandten Teil des zweiteiligen Einlasskanals (400, 400') und des zweiteiligen Auslasskanals (410, 410') fluidleitend verschließt oder verschließbar macht,
wobei die Mischeinheit (200, 200') und das Reservoir (300, 300') reversibel über einen ersten Formschluss (430) miteinander verbunden oder verbindbar sind
**dadurch gekennzeichnet, dass** das Verschlusselement (450) nach einem Trennen des Reservoirs (300) von der Mischeinheit (200) durch Lösen des ersten Formschlusses (430) in eine Verschlussposition verbringbar ist, um den der Mischeinheit (200) zugewandten Teil des zweiteiligen Einlasskanals (400) und des zweiteiligen Auslasskanals (410) fluidleitend zu verschließen.

3. Vorrichtung (100, 100') nach Anspruch 1 oder 2, **wobei** das Reservoir (300, 300') einen Reservoirbehälter (310, 310'), in dem mindestens eine fluidleitend geschlossene Ampulle (330, 330') mit einem Ampullenkörper (331, 331') und einem Ampullenkopf (332, 332') angeordnet ist und in der Ampulle (330, 330') die Monomerflüssigkeit (510, 510') lagert, und einen Hohlraum (340, 340') im Bereich des Ampullenkopfs (332, 332') aufweist, wobei der Hohlraum (340, 340') fluidleitend mit dem Einlasskanal (400, 400') verbunden ist und eine Verbindung (350, 350') zur Ampulle (330, 330') umfasst, wobei der Ampullenkopf (332, 332') zumindest bereichsweise in der Verbindung (350, 350') angeordnet ist und der Reservoirbehälter (310, 310') zumindest abschnittsweise einen verformbaren Bereich (320, 320') umfasst, so dass ein Verkippen der Ampulle (330, 330') um einen Drehpunkt (420) gegen die Verbindung (350, 350') ermöglicht ist.

4. Vorrichtung (100, 100') nach Anspruch 3, **wobei** der Einlasskanal (400, 400') einen geringeren Abstand zum Drehpunkt (420) aufweist als der Auslasskanal (410, 410').

5. Vorrichtung (100, 100') nach einem der vorhergehenden Ansprüche, **wobei** der Auslasskanal (410, 410') proximal zum Einlasskanal (400, 400') in den Innenraum (215, 215') mündet.

6. Vorrichtung (100, 100') nach einem der vorhergehenden Ansprüche, **wobei** der Einlasskanal (400, 400') an einem der Mischeinheit (200, 200') gegenüberliegendem Einlasskanalende als Trichter ausgebildet ist.

7. Vorrichtung (100, 100') nach einem der vorhergehenden Ansprüche, **wobei** der Auslasskanal (410, 410') einen minimalen Auslasskanaldurchmesser (411, 411') aufweist, welcher mindestens einem halben minimalen Einlasskanaldurchmessers (401, 401') des Einlasskanals (400, 400') entspricht.

8. Vorrichtung (100, 100') nach Anspruch 1, **wobei** die Mischeinheit (200, 200') und das Reservoir (300, 300') reversibel über einen ersten Formschluss (430) miteinander verbunden oder verbindbar sind.

9. Vorrichtung (100) nach einem der Ansprüche 2 bis 7, **wobei** das Verschlusselement (450) eine Schraube ist.

10. Vorrichtung (100, 100') nach einem der Ansprüche 2 bis 8, **wobei** die Mischeinheit (200, 200') und das Reservoir (300, 300') reversibel über einen zweiten Formschluss (440) miteinander verbunden oder verbindbar sind.

11. Verfahren (600) zum Bereitstellen eines Knochenzementteigs (520) aus zwei Ausgangskomponenten mittels einer Vorrichtung (100, 100') nach einem der vorhergehenden Ansprüche, umfassend die Schritte:
a. Fließen (610) der Monomerflüssigkeit (510, 510') aus dem Reservoir (300, 300') durch den Einlasskanal (400, 400') in den distalen Teil (217, 217') des Innenraums (215, 215') unter gleichzeitigem Ausleiten eines Gases (530, 530') aus dem Innenraum (215, 215') durch den Auslasskanal (410, 410') in das Reservoir (300, 300'),
b. Fördern (620) der Monomerflüssigkeit (510, 510') aus dem distalen Teil (217, 217') des Innenraums (215, 215') durch das Leitungsmittel (230, 230') in den proximalen Teil (216, 216') des Innenraums (215, 215') mittels eines Vortreibens des Förderkolbens (240, 240') in Richtung des Austragskolben (220, 220').

12. Verfahren (600) nach Anspruch 11 mittels einer Vorrichtung (100, 100') nach einem der Ansprüche 1 bis 10, **wobei** vor dem Fördern (610) der Monomerflüssigkeit (510, 510') in Schritt b. der der Mischeinheit (200, 200') zugewandte Teil des zweiteiligen Einlasskanals (400, 400') und des zweiteiligen Auslasskanals (410, 410') durch das Verschlusselement (450, 450') fluidleitend verschlossen (615) wird.

## Claims

1. A device (100, 100') for preparing a bone cement paste (520) from two starting components, the device comprising
a mixing unit (200, 200') comprising a hollow cylindrical cartridge (210, 210') with an interior space (215, 215'), wherein a discharge piston (220, 220') which is axially movable in the interior space (215, 215') is arranged in the interior space (215, 215'), which discharge piston divides the interior space (215, 215') into a proximal part (216, 216') of the interior space (215, 215') and a distal part (217, 217') of the interior space (215, 215'), wherein the proximal part (216, 216') and the distal part (217, 217') of the interior space (215, 215') are fluidically connected to one another via a conduit means (230, 230'), wherein a bone cement powder (500, 500') is stored as the first starting component in the proximal part (216, 216') of the interior space (215, 215'), and wherein a delivery piston (240, 240') which is axially movable in the interior space (215, 215') is arranged in the distal part (217, 217') of the interior space (215, 215'),
and a reservoir (300, 300') for a monomer liquid (510, 510') as the second starting component, which reservoir is or can be fluidically connected via an inlet channel (400, 400') to the distal part (217, 217') of the interior space (215, 215') in order to introduce the monomer liquid (510, 510') from the reservoir (300, 300') into the mixing unit (200, 200'), wherein the reservoir (300, 300') and the mixing unit (200, 200') are or can be fluidically connected via an outlet channel (410, 410'), via which a gas (530, 530') can be discharged from the interior space (215, 215') into the reservoir (300, 300'),
wherein the inlet channel (400, 400') and the outlet channel (410, 410') are formed at least in two parts, so that the mixing unit (200, 200') and the reservoir (300, 300') are fluidically connected to one another in a first channel position of the inlet channel (400, 400') and the outlet channel (410, 410') and are fluidically separated from one another in a second channel position of the inlet channel (400, 400') and the outlet channel (410, 410'), wherein the device (100, 100') comprises a closure element (450, 450') which fluidically closes or makes closable at least a part of the two-part inlet channel (400, 400') and the two-part outlet channel (410, 410') that faces the mixing unit (200, 200'),
**characterized in that** the closure element (450') is a rotary valve through which the part of the two-part inlet channel (400') and the two-part outlet channel (410') that faces the mixing unit (200') extends and which, in a first rotary valve position, leaves the inlet channel (400) and the outlet channel (410') in the first channel position and, by rotating into a second rotary valve position, moves the inlet channel (400') and the outlet channel (410') into the second channel position.

2. A device (100, 100') for preparing a bone cement paste (520) from two starting components, the device comprising
a mixing unit (200, 200') comprising a hollow cylindrical cartridge (210, 210') with an interior space (215, 215'), wherein a discharge piston (220, 220') which is axially movable in the interior space (215, 215') is arranged in the interior space (215, 215'), which discharge piston divides the interior space (215, 215') into a proximal part (216, 216') of the interior space (215, 215') and a distal part (217, 217') of the interior space (215, 215'), wherein the proximal part (216, 216') and the distal part (217, 217') of the interior space (215, 215') are fluidically connected to one another via a conduit means (230, 230'),
wherein a bone cement powder (500, 500') is stored as the first starting component in the proximal part (216, 216') of the interior space (215, 215'), and wherein a delivery piston (240, 240') which is axially movable in the interior space (215, 215') is arranged in the distal part (217, 217') of the interior space (215, 215'),
and a reservoir (300, 300') for a monomer liquid (510, 510') as the second starting component, which reservoir is or can be fluidically connected via an inlet channel (400, 400') to the distal part (217, 217') of the interior space (215, 215') in order to introduce the monomer liquid (510, 510') from the reservoir (300, 300') into the mixing unit (200, 200'), wherein the reservoir (300, 300') and the mixing unit (200, 200') are or can be fluidically connected via an outlet channel (410, 410'), via which a gas (530, 530') can be discharged from the interior space (215, 215') into the reservoir (300, 300'),
wherein the inlet channel (400, 400') and the outlet channel (410, 410') are formed at least in two parts, so that the mixing unit (200, 200') and the reservoir (300, 300') are fluidically connected to one another in a first channel position of the inlet channel (400, 400') and the outlet channel (410, 410') and are fluidically separated from one another in a second channel position of the inlet channel (400, 400') and the outlet channel (410, 410'),
wherein the device (100, 100') comprises a closure element (450, 450') which fluidically closes or makes closable at least a part of the two-part inlet channel (400, 400') and the two-part outlet channel (410, 410') that faces the mixing unit (200, 200'),
wherein the mixing unit (200, 200') and the reservoir (300, 300') are or can be reversibly connected to one another via a first form fit (430),
**characterized in that** the closure element (450) can be brought into a closure position after separation of the reservoir (300) from the mixing unit (200) by releasing the first form fit (430), in order to fluidically close the part of the two-part inlet channel (400) and the two-part outlet channel (410) that faces the mixing unit (200).

3. The device (100, 100') according to claim 1 or 2, **wherein** the reservoir (300, 300') comprises a reservoir container (310, 310') in which at least one fluidically closed ampule (330, 330') with an ampule body (331, 331') and an ampule head (332, 332') is arranged, and the monomer liquid (510, 510') is stored in the ampule (330, 330'), and comprises a cavity (340, 340') in the region of the ampule head (332, 332'), wherein the cavity (340, 340') is fluidically connected to the inlet channel (400, 400') and comprises a connection (350, 350') to the ampule (330, 330'), wherein the ampule head (332, 332') is arranged at least partially in the connection (350, 350'), and the reservoir container (310, 310') comprises a deformable region (320, 320') at least in portions, so that tilting of the ampule (330, 330') about a pivot point (420) against the connection (350, 350') is possible.

4. The device (100, 100') according to claim 3, **wherein** the inlet channel (400, 400') has a smaller distance from the pivot point (420) than the outlet channel (410, 410').

5. The device (100, 100') according to any of the preceding claims, **wherein** the outlet channel (410, 410') opens into the interior space (215, 215') proximally to the inlet channel (400, 400').

6. The device (100, 100') according to any of the preceding claims, **wherein** the inlet channel (400, 400') is designed as a funnel at an inlet channel end opposite the mixing unit (200, 200').

7. The device (100, 100') according to any of the preceding claims, **wherein** the outlet channel (410, 410') has a minimum outlet channel diameter (411, 411') which corresponds to at least half the minimum inlet channel diameter (401, 401') of the inlet channel (400, 400').

8. The device (100, 100') according to claim 1, **wherein** the mixing unit (200, 200') and the reservoir (300, 300') are or can be reversibly connected to one another via a first form fit (430).

9. The device (100) according to any of claims 2 to 7, **wherein** the closure element (450) is a screw.

10. The device (100, 100') according to any of claims 2 to 8, **wherein** the mixing unit (200, 200') and the reservoir (300, 300') are or can be reversibly connected to one another via a second form fit (440).

11. A method (600) for preparing a bone cement paste (520) from two starting components by means of a device (100, 100') according to any of the preceding claims, comprising the steps of:
a. the monomer liquid (510, 510') flowing (610) from the reservoir (300, 300') through the inlet channel (400, 400') into the distal part (217, 217') of the interior space (215, 215') while a gas (530, 530') is simultaneously discharged from the interior space (215, 215') through the outlet channel (410, 410') into the reservoir (300, 300'),
b. the monomer liquid (510, 510') being conveyed (620) from the distal part (217, 217') of the interior space (215, 215') through the conduit means (230, 230') into the proximal part (216, 216') of the interior space (215, 215') by means of advancement of the delivery piston (240, 240') in the direction of the discharge piston (220, 220').

12. The method (600) according to claim 11 by means of a device (100, 100') according to any of claims 1 to 10, **wherein,** before the conveying (610) of the monomer liquid (510, 510') in step b., the part of the two-part inlet channel (400, 400') and the two-part outlet channel (410, 410') that faces the mixing unit (200, 200') is fluidically closed (615) by the closure element (450, 450').

## Revendications

1. Dispositif (100, 100') permettant de fournir une pâte de ciment osseux (520) à partir de deux composants de départ, comprenant
une unité de mélange (200, 200') présentant une cartouche (210, 210') en forme de cylindre creux comportant un espace intérieur (215, 215'), dans lequel un piston de distribution (220, 220') mobile axialement dans l'espace intérieur (215, 215') est disposé dans l'espace intérieur (215, 215'), lequel piston de distribution divise l'espace intérieur (215, 215') en une partie proximale (216, 216') de l'espace intérieur (215, 215') et en une partie distale (217, 217') de l'espace intérieur (215, 215'), dans lequel la partie proximale (216, 216') et la partie distale (217, 217') de l'espace intérieur (215, 215') sont raccordées l'une à l'autre d'une manière conductrice de fluide par l'intermédiaire d'un moyen de conduite (230, 230'),
dans lequel une poudre de ciment osseux (500, 500') est stockée dans la partie proximale (216, 216') de l'espace intérieur (215, 215') comme premier composant de départ et dans lequel un piston de transport (240, 240') mobile axialement dans l'espace intérieur (215, 215') est disposé dans la partie distale (217, 217') de l'espace intérieur (215, 215'),
et un réservoir (300, 300') pour un liquide monomère (510, 510') comme second composant de départ, lequel réservoir est raccordé ou peut être raccordé d'une manière conductrice de fluide à la partie distale (217, 217') de l'espace intérieur (215, 215') par l'intermédiaire d'un canal d'entrée (400, 400') pour l'introduction du liquide monomère (510, 510') provenant du réservoir (300, 300') dans l'unité de mélange (200, 200'),
dans lequel le réservoir (300, 300') et l'unité de mélange (200, 200') sont raccordés ou peuvent être raccordés d'une manière conductrice de fluide par l'intermédiaire d'un canal de sortie (410, 410') par l'intermédiaire duquel un gaz (530, 530') peut être évacué depuis l'espace intérieur (215, 215') dans le réservoir (300, 300'),
dans lequel le canal d'entrée (400, 400') et le canal de sortie (410, 410') sont formés au moins en deux parties, de sorte que l'unité de mélange (200, 200') et le réservoir (300, 300') sont raccordés l'un à l'autre d'une manière conductrice de fluide dans une première position de canal du canal d'entrée (400, 400') et du canal de sortie (410, 410') et sont séparés l'un de l'autre d'une manière conductrice de fluide dans une seconde position de canal du canal d'entrée (400, 400') et du canal de sortie (410, 410'),
dans lequel le dispositif (100, 100') présente un élément de fermeture (450, 450') qui ferme ou rend fermable d'une manière conductrice de fluide au moins une partie, tournée vers l'unité de mélange (200, 200'), du canal d'entrée (400, 400') en deux parties et du canal de sortie (410, 410') en deux parties,
**caractérisé en ce que** l'élément de fermeture (450') est une vanne rotative à travers laquelle s'étendent les parties du canal d'entrée (400') en deux parties et du canal de sortie (410') en deux parties tournées vers l'unité de mélange (200') et qui, dans une première position de vanne rotative, laisse le canal d'entrée (400) et le canal de sortie (410') dans la première position de canal et, par une rotation dans une seconde position de vanne rotative, amène le canal d'entrée (400') et le canal de sortie (410') dans la seconde position de canal.

2. Dispositif (100, 100') permettant de fournir une pâte de ciment osseux (520) à partir de deux composants de départ, comprenant
une unité de mélange (200, 200') présentant une cartouche (210, 210') en forme de cylindre creux comportant un espace intérieur (215, 215'), dans lequel un piston de distribution (220, 220') mobile axialement dans l'espace intérieur (215, 215') est disposé dans l'espace intérieur (215, 215'), lequel piston de distribution divise l'espace intérieur (215, 215') en une partie proximale (216, 216') de l'espace intérieur (215, 215') et en une partie distale (217, 217') de l'espace intérieur (215, 215'), dans lequel la partie proximale (216, 216') et la partie distale (217, 217') de l'espace intérieur (215, 215') sont raccordées l'une à l'autre d'une manière conductrice de fluide par l'intermédiaire d'un moyen de conduite (230, 230'),
dans lequel une poudre de ciment osseux (500, 500') est stockée dans la partie proximale (216, 216') de l'espace intérieur (215, 215') comme premier composant de départ et dans lequel un piston de transport (240, 240') mobile axialement dans l'espace intérieur (215, 215') est disposé dans la partie distale (217, 217') de l'espace intérieur (215, 215'),
et un réservoir (300, 300') pour un liquide monomère (510, 510') comme second composant de départ, lequel réservoir est raccordé ou peut être raccordé d'une manière conductrice de fluide à la partie distale (217, 217') de l'espace intérieur (215, 215') par l'intermédiaire d'un canal d'entrée (400, 400') pour l'introduction du liquide monomère (510, 510') provenant du réservoir (300, 300') dans l'unité de mélange (200, 200'),
dans lequel le réservoir (300, 300') et l'unité de mélange (200, 200') sont raccordés ou peuvent être raccordés d'une manière conductrice de fluide par l'intermédiaire d'un canal de sortie (410, 410') par l'intermédiaire duquel un gaz (530, 530') peut être évacué depuis l'espace intérieur (215, 215') dans le réservoir (300, 300'),
dans lequel le canal d'entrée (400, 400') et le canal de sortie (410, 410') sont formés au moins en deux parties, de sorte que l'unité de mélange (200, 200') et le réservoir (300, 300') sont raccordés l'un à l'autre d'une manière conductrice de fluide dans une première position de canal du canal d'entrée (400, 400') et du canal de sortie (410, 410') et sont séparés l'un de l'autre d'une manière conductrice de fluide dans une seconde position de canal du canal d'entrée (400, 400') et du canal de sortie (410, 410'),
dans lequel le dispositif (100, 100') présente un élément de fermeture (450, 450') qui ferme ou rend fermable d'une manière conductrice de fluide au moins une partie, tournée vers l'unité de mélange (200, 200'), du canal d'entrée (400, 400') en deux parties et du canal de sortie (410, 410') en deux parties,
dans lequel l'unité de mélange (200, 200') et le réservoir (300, 300') sont reliés ou peuvent être reliés l'un à l'autre de manière réversible par l'intermédiaire d'une première liaison par complémentarité de forme (430),
**caractérisé en ce que** l'élément de fermeture (450) peut être amené dans une position de fermeture après une séparation du réservoir (300) de l'unité de mélange (200) en libérant la première liaison par complémentarité de forme (430) afin de fermer d'une manière conductrice de fluide les parties du canal d'entrée (400) en deux parties et du canal de sortie (410) en deux parties tournées vers l'unité de mélange (200).

3. Dispositif (100, 100') selon la revendication 1 ou 2, dans lequel le réservoir (300, 300') présente un récipient de réservoir (310, 310') dans lequel est disposée au moins une ampoule (330, 330') fermée d'une manière conductrice de fluide et comportant un corps d'ampoule (331, 331') et une tête d'ampoule (332, 332'), et le liquide monomère (510, 510') est stocké dans l'ampoule (330, 330'), et le réservoir présente un espace creux (340, 340') dans la zone de la tête d'ampoule (332, 332'), dans lequel l'espace creux (340, 340') est raccordé d'une manière conductrice de fluide au canal d'entrée (400, 400') et comprend une région de liaison (350, 350') vers l'ampoule (330, 330'), dans lequel la tête d'ampoule (332, 332') est disposée, au moins dans certaines zones, dans la région de liaison (350, 350') et le récipient de réservoir (310, 310') comprend, au moins dans certaines sections, une zone déformable (320, 320'), de sorte qu'un basculement de l'ampoule (330, 330') autour d'un point de rotation (420) contre la région de liaison (350, 350') est possible.

4. Dispositif (100, 100') selon la revendication 3, dans lequel le canal d'entrée (400, 400') présente une distance par rapport au point de rotation (420) inférieure à celle du canal de sortie (410, 410') par rapport audit point de rotation.

5. Dispositif (100, 100') selon l'une des revendications précédentes, dans lequel le canal de sortie (410, 410') débouche dans l'espace intérieur (215, 215') de manière proximale par rapport au canal d'entrée (400, 400').

6. Dispositif (100, 100') selon l'une des revendications précédentes, dans lequel le canal d'entrée (400, 400') est réalisé sous forme d'entonnoir à une extrémité de canal d'entrée opposée à l'unité de mélange (200, 200').

7. Dispositif (100, 100') selon l'une des revendications précédentes, dans lequel le canal de sortie (410, 410') présente un diamètre minimal de canal de sortie (411, 411') correspondant à au moins un demi-diamètre minimal de canal d'entrée (401, 401') du canal d'entrée (400, 400').

8. Dispositif (100, 100') selon la revendication 1, dans lequel l'unité de mélange (200, 200') et le réservoir (300, 300') sont reliés ou peuvent être reliés l'un à l'autre de manière réversible par l'intermédiaire d'une première liaison par complémentarité de forme (430).

9. Dispositif (100) selon l'une des revendications 2 à 7, dans lequel l'élément de fermeture (450) est une vis.

10. Dispositif (100, 100') selon l'une des revendications 2 à 8, dans lequel l'unité de mélange (200, 200') et le réservoir (300, 300') sont reliés ou peuvent être reliés l'un à l'autre de manière réversible par l'intermédiaire d'une seconde liaison par complémentarité de forme (440).

11. Procédé (600) permettant de fournir une pâte de ciment osseux (520) à partir de deux composants de départ à l'aide d'un dispositif (100, 100') selon l'une des revendications précédentes, comprenant les étapes consistant à :
a. faire s'écouler (610) le liquide monomère (510, 510') hors du réservoir (300, 300'), à travers le canal d'entrée (400, 400') et dans la partie distale (217, 217') de l'espace intérieur (215, 215') tout en évacuant simultanément un gaz (530, 530') hors de l'espace intérieur (215, 215'), à travers le canal de sortie (410, 410') et dans le réservoir (300, 300'),
b. transporter (620) le liquide monomère (510, 510') depuis la partie distale (217, 217') de l'espace intérieur (215, 215'), à travers le moyen de conduite (230, 230') et dans la partie proximale (216, 216') de l'espace intérieur (215, 215') au moyen d'une propulsion du piston de transport (240, 240') en direction du piston de distribution (220, 220').

12. Procédé (600) selon la revendication 11 au moyen d'un dispositif (100, 100') selon l'une des revendications 1 à 10, dans lequel, avant le transport (610) du liquide monomère (510, 510') à l'étape b., les parties du canal d'entrée (400, 400') en deux parties et du canal de sortie (410, 410') en deux parties tournées vers l'unité de mélange (200, 200') sont fermées (615) d'une manière conductrice de fluide par l'élément de fermeture (450, 450').
